# NEW EUROPEAN PATENT SPECIFICATION

(11) **EP 2 400 954 B2**
(45) Date of publication and mention of the opposition decision: **26.06.2024**
(45) Mention of the grant of the patent: 28.09.2016
(21) Application number: 10705984.2
(22) Date of filing: 01.03.2010
(51) Int. Cl.: A61K 9/20, A61K 9/28, A61K 31/439

(54) **PROCESS FOR FORMING SOLID ORAL DOSAGE FORMS OF SOLIFENACIN SUCCINATE**
VERFAHREN ZUR BILDUNG FESTER ORALER DOSIERFORMEN VON SOLIFENACIN SUCCINATE
PROCÉDÉ DE FABRICATION DE FORMES POSOLOGIQUES ORALES DE SOLIFÉNACINE SUCCINATE

(30) Priority: 27.02.2009 SI 200900056; 08.06.2009 SI 200900159
(43) Date of publication of application: 04.01.2012
(73) Proprietor: KRKA, d.d., Novo mesto, 8501 Novo mesto (SI)
(72) Inventor: VRBINC, Miha, 8000 Novo mesto (SI); VRECER, Franc, 8351 Straza (SI); TURK, Urska, 8000 Novo mesto (SI); JURSIC, Urska, 1000 Ljublijana (SI)
(74) Representative: Hoefer & Partner Patentanwälte mbB
(86) International application number: PCT/EP2010/001262
(87) International publication number: WO 2010/097243

(56) References cited:
- EP-A1- 1 728 791
- WO-A2-2007/072169
- WO-A2-2008/128028
- WO-A2-2010/012459
- US-A1- 2008 114 028

## Description

### TECHNICAL FIELD

The subject of the invention includes pharmaceutical compositions comprising solifenacin succinate as the active pharmaceutical ingredient prepared by dry technological methods, preferably by the direct compression or roller compaction method, i.e. by the process in the absence of a liquid solvent.

### BACKGROUND OF THE INVENTION

(3R)-1-azabicyclo[2.2.2]oct-3-yl-(1S)-1-phenyl-3,4-dihydroisoquinoline- 2-(1H)-carboxylate or (S)-phenyl-1,2,3,4-tetrahydroisoquinoline-2-carboxylic acid 3(R)-quinuclidinyl ester having the following formula: 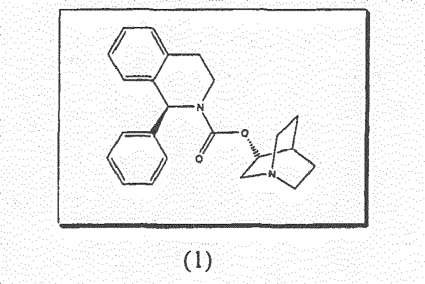 is known as solifenacin, also known as YM-905 (in its free base form) and YM-67905 (in its succinate form). Solifenacin has the molecular formula C₂₃H₂₆O₂, and a molecular weight of 362.465.

One of the solifenacin salts is solifenacin succinate, which is a urinary antispasmodic, acting as a selective antagonist to the M(3)-receptor. It is used for treatment of symptoms of overactive bladder ("OAB"), such as urinary urgency and increased urinary frequency, as may occur in patients with overactive bladder syndrome, as reviewed in Chilman-Blair et al., Solifenacin: Treatment of overactive bladder, Drugs of Today, 40(4): 343 - 353 (2004).

The commercial solifenacin tablet formulation is marketed under the trade name VESIcare^{®}. VESIcare^{®} was approved by the FDA for once daily treatment of OAB and is prescribed as 5 mg and 10 mg film coated tablets.

The technical problem to be solved was to improve the chemical stability and dissolution profiles of solifenacin succinate, and also to improve the technological parameters important for the preparation of solid oral dosage forms. There is a wish to prepare film coated tablets by a robust and reproducible technological method by selection of excipients that allow the preparation of the film coated tablet by a process in the absence of a liquid solvent.

The problem which occurs is the stability of solifenacin and its salts in pharmaceutical compositions. This was described in detail in patent application EP 1728791. The solution, which is claimed therein, is the control of process parameters as not to obtain a too high weight ratio of the amorphous fraction of solifenacin succinate which combined with a too high humidity leads to the chemical degradation of the active ingredient. The problem of this approach is that the parameter range wherein tablets of the required quality are obtained is very narrow and thus difficult to achieve in industrial scale.

On the other hand, it is reported that direct tableting is disadvantageous due to the strong aggregation properties of solifenacin and its salts leading to poor content uniformity and sticking of the mixture to the punches during compression. It has been explained that high ratios of amorphous form lead to stability problems which results in high amount of the main degradation product N-oxide of solifenacin generated.

In the international publication of patent application WO 2008/128028, a pharmaceutical composition was disclosed prepared by dissolving solifenacin succinate with one or more water soluble excipients and optionally an antioxidant, wherein the solvent was either rapidly evaporated, or a co-precipitate was obtained by adding an anti-solvent. The application further discloses processes including contacting a solution of solifenacin succinate with an undissolved excipient (adsorption of solifenacin succinate on Zeopharm 600 or Fujicalin), the formation of resinates or co-precipitates with cyclodextrins.

US 2008/0114028 A1 describes that polymorphic forms of solifenacin have been prepared and characterized. These polymorphic forms are considered in this document as being useful in pharmaceutical compositions. In particular, this document mentions the use of at least one of an amorphous form of solifenacin succinate and a crystalline form of solifenacin succinate characterized by PXRD peaks at about 4.3, 14.7, and 16.2°±0.2° 2θ in the manufacture of a medicament for the treatment of overactive bladder syndrome.

WO 2007/072169 A2 relates to the combined use of a PDE5 inhibitor and a muscarinic antagonist in the treatment of lower urinary tract symptoms (LUTS), such as urgency, frequency, nocturia and urge incontinence.

WO 2010/012459 A2 (International publication date: 04.02.2010) relates to the synthesis of solifenacin, the preparation of its salts and their inclusion into pharmaceutically acceptable dosage forms. In particular, a preparation of a tablet by direct compression is described in an Example provided in WO 2010/012459 A2.

WO 2009/012987 describes pharmaceutical compositions comprising solifenacin or pharmaceutically acceptable salts thereof substantially in amorphous form, characterized in that the composition comprises an amorphous solifenacin succinate which has first been stabilized with a stabilizer such as PEG, HPMC, MC, PVP etc. WO2009/012987 also reports that the crystalline form of solifenacin salts shows conversion to amorphous form and that this conversion leads to chemical degradation of the drug. On the other hand, the amorphous form would be very desirable due to a higher bioavailability.

It was now surprisingly found out how the problems observed with direct compression, such as poor uniformity of content and sticking to the punches can be omitted, while at the same time providing a pharmaceutical formulation of solifenacin or its salts with high bioavailability, less prone to degradation, and with excellent uniformity.

### DETAILED DESCRIPTION OF THE INVENTION

The objective of the present invention is to develop an improved method of preparing stable pharmaceutical compositions comprising solifenacin or its pharmaceutically acceptable salts as the active pharmaceutical ingredient and whose manufacturing process is simple, straightforward, and may be conducted using standard formulation methods and excipients.

It has been found out that manufacturing solid dosage forms such as tablets containing solifenacin or its pharmaceutically acceptable salts by using wet granulation, especially aqueous granulation described in the prior art, documents can result in increased content of degradation products (such as hydrolytic and/or oxidative degradation product of solifenacin or its pharmaceutically acceptable salts) due to the presence of increased moisture and heat/temperature. Partial amorphisation of solifenacin or its pharmaceutically acceptable salts can be obtained by the aqueous granulation due to partial dissolution of solifenacin or its pharmaceutically acceptable salts during granulation with subsequent fast evaporation of the solvent at subsequent drying. The presence of solifenacin or its pharmaceutically acceptable salts in partial or complete amorphous state, can result in decreased stability (physical and/or chemical) of solifenacin or its pharmaceutically acceptable salts in the pharmaceutical composition.

It has been surprisingly found out that a stable pharmaceutical product with excellent content uniformity can be manufactured by the present invention i.e. by the manufacturing processes in the absence of a solvent.

According to one aspect, the present invention provides a process for the preparation of a solid oral dosage form as defined in claim 1.

According to a further aspect, the present invention provides a solid oral dosage form as defined in claim 8.

There is provided a process of forming a solid oral dosage form comprising
a.) an effective amount of crystalline solifenacin succinate,
b.) pharmaceutically acceptable additives suitable for the preparation of solid oral dosage forms,
in the absence of a solvent, e.g. the solid
oral dosage form can be prepared by a dry technological method, such as direct compression, compaction or dry granulation.

In one of the preferred embodiments of the present invention, there is provided a process of making a solid dosage form, comprising the steps of:
i) optionally grinding crystalline solifenacin succinate and pharmaceutically acceptable excipients,
ii) subjecting a mixture, preferably a homogenous mixture, of the optionally ground active ingredient and excipients to compression to form a comprimate,
iii) converting the comprimate to form a granulate and
iv) compressing/tabletting the granulate to form the solid oral dosage form.

An alternative step iv) may further comprise the addition of a second part of the active compound and/ or excipients prior to the compression/tabletting.

The process i) to iv) is performed in the absence of a solvent, such as for example water, methanol, ethanol, e.g. the solid oral dosage form and/or the granulate and/or the comprimate is prepared by a dry technological method, such as direct compression, compaction or dry granulation. The process may be carried out under ambient conditions of temperature and ambient conditions of humidity; it is not necessary to ensure that the process is carried out in a dry atmosphere.

In the context of the present application, the term "ambient conditions of temperature" can for example mean a temperature or a temperature range selected from -10°C to 45 °C, preferably selected from 5°C to 35 °C, more preferably selected from 15°C to 30 °C, still more preferably selected from 18°C to 25 °C, especially 20°C.

In the context of the present application, the term "under ambient conditions of humidity" can for example mean under an atmosphere, in particular air, having a content of water of less than 50 g per m³ atmosphere, preferably of less than 25 g per m³ atmosphere, more preferably of less than 21 g per m³ atmosphere, still more preferably of less than 12 g per m³ atmosphere, especially preferably of less than 10 g per m³ atmosphere, in particular of less than 5 g per m³ atmosphere, on the basis of an atmosphere being present at standard pressure, in particular at a pressure of 101 325 Pa.

According to a preferred embodiment, the term "in the absence of solvent" has the meaning of in the absence of solvent comprising or consisting of one or more of water, dioxane, toluene, acetonitrile, tetrahydrofurane, ethyl acetate, ethanol, methanol and mixtures thereof.

According to the invention the process steps i) to iv) are performed in the absence of a solvent, such as water, methanol, ethanol, in particular by a dry technological method, such as direct compression, compaction or dry granulation. In particular, the complete sequence of process steps i) to iv) and any intermediate steps, more preferably the complete sequence of process steps i) to iv) of a process of forming a solid oral dosage form, are carried out in the absence of solvent.

According to an embodiment of the invention, the term "in the absence of water" means that during the preparation process, e.g. of a solid dosage form or a mixture to be compressed, no water has been (is) added as such. According to a preferred embodiment, no water is added (as a solvent) during the preparation process, i.e. it is a dry technological process.

In a further embodiment of the invention, the total amount of water present in an ingredient to be used in a process step mentioned herein and/or in a sequence of process steps mentioned herein, may be chosen to be below 4 weight%, preferably below 3.5 weight% and more preferably below 3 weight%, each based on the total weight of the ingredient.

In a further embodiment of the invention, the total amount of water present in an intermediate product or combination or mixture of ingredients, in particular granulate and/or comprimate, present and/or resulting from an process step mentioned herein and/or a combination of said process steps, may be chosen to be below 4 weight%, preferably below 3.5 weight% and more preferably below 3 weight%, each based on the total weight of the respective intermediate product (or combination or mixture of ingredients).

In a further embodiment of the invention, the total amount of water of the solid oral dosage form may be chosen to be below 4 weight%, preferably below 3.5 weight% and more preferably below 3 weight%, each based on the total weight of the solid oral dosage form.

The initial grinding step i) may be carried out according to conventional milling methods or micronisation methods.

Particles of this size are obtained by methods as grinding in an air jet mill, hammer and screen mill, fine impact mill, ball mill or vibrator mill.

Micronisation is preferably effected by known methods using an ultrasonic disintegrator, e.g. of the BRANSON Sonifier type, or by stirring a suspension with a high speed agitator, for example with a stirrer of the HOMOREX type.

The ground particles may optionally be sieved and mixed according to the known methods which are pharmaceutically acceptable. Compaction may be carried out using a slugging or roller compaction technique. The preferred method of dry granulation is roller compaction. Roller compaction is conventional and essentially utilizes two rollers which roll towards each other. A hydraulic ram forces one of the rollers against the other roller to exert a compacting force against the ground particles fed into the roller compactor via a screw conveyor system. A compaction force of minimal 10 kN is preferably used. Above that value of compaction force, it has been surprisingly found that the solid dosage form is obtained by preparation granulate, which disintegrates into discrete primary particles at a desirable rate. The minimal roller speed during roller compaction is preferably 1 rpm.

The comprimate resembles a thin ribbon in segments, depending on the surface of the rollers. The comprimate may be milled and/or screened to produce the granulate. Screening involves the passing of the comprimate using an oscillating mill. It has been found that the granulate emerging from the roller compactor after screening and/or milling can in fact be tabletted to form a solid oral dosage forms without affecting the properties of the solid dosage form.

Step i) may be omitted by the selection of the suitable particle size of both active ingredient and excipients, which exclude grinding as the initial step in the pharmaceutical technological process of preparation.

In another preferred embodiment, the process of forming a solid oral dosage form is direct tabletting, which comprises the following steps:
a) mixing crystalline solifenacin succinate with pharmaceutically acceptable excipients, b) compressing/tabletting of the mixed active ingredient and pharmaceutically acceptable excipients to form a solid dosage form.

Mixing under step a) can optionally be performed stepwise, meaning that at first active ingredient is mixed with one or only a part of excipients and the rest of excipients are added in second phase of mixing.

According to an embodiment of the invention the process steps a) to b) are performed in the absence of a solvent, such as water, methanol, ethanol, in particular by a dry technological method, such as direct compression, compaction or dry granulation. In particular, the complete sequence of process steps a) to b) and any intermediate steps, more preferably the complete sequence of process steps of a process of forming a solid oral dosage form, are carried out in the absence of solvent.

A solid oral dosage form according to the invention comprises additives conventional in the dosage form in question. Tabletting aids, commonly used in tablet formulation can be used and reference is made to the extensive literature on the subject. These include but are not limited to disintegrants, binders, lubricants, stabilising agents (e.g. antioxidants), diluents/fillers, surfactants and the like.

As disintegrants and/or superdisintegrants, one can particularly mention starches (e.g. maize starch, wheat starch, potato starch), modified starches (sodium starch glycolate, pregelatinised starch), microcrystalline cellulose, low-substituted hydroxypropylcellulose (comprising from 5 to 16% by weight of hydroxypropyl groups), CMC-Ca, CMC-Na, crosslinked CMC-Na (e.g. Ac-Di-Sol, Explocel, Nymcel ZSX, Pharmacel XL, Primellose, Solutab, Vivasol), crospovidone (Kollidon CL, Kollidon CL-F, Kollidon CL-SF, Kollidon CL-M, Polyplasdone XL, Polyplasdone XL-10, Polyplasdone INF-10), alginic acid, sodium alginate, guar gum, gellan gum, Xanthan SM, polacrilin potassium, or any mixtures thereof. The volume average particle size (diameter) of disintegrant determined by laser diffraction method can be in the range 1 to 200 µm, preferably 3 to 150 µm. The particle size of disintegrant influence the rate and the extent of disintegration of tabltes.

As binders one can particularly mention starches (e.g. maize starch, wheat starch, potato starch), modified starches (sodium starch glycolate, pregelatinised starch), polymethacrylate, microcrystalline cellulose, cellulose derivatives (hydroxyethylcellulose, hydroxypropylcellulose, hydroxypropylmethylcellulose, low-substituted hydroxypropylcellulose (comprising from 5 to 16% by weight of hydroxypropyl groups) or any other cellulose ether, graft copolymer of polyvinyl alcohol with polyethylene glycol or any mixture thereof. The graft copolymer of polyvinyl alcohol with polyethylene glycol present in the pharmaceutical composition according to the present invention has a melting point of between 150°C and 300°C, preferably between 180°C and 250°C, more preferably between 200°C and 220°C.

The graft copolymer of polyvinyl alcohol with polyethylene glycol present in the pharmaceutical composition according to a preferred embodiment of the present invention has hydrophilic properties. Preferably, it is the known copolymer under the trade name of Kollicoat^{®} IR comprising 75% polyvinyl alcohol residues and 25% polyethylene glycol residues. The molecular weight of the copolymer is approximately 45,000 Daltons with a melting point of approximately 209°C. Kollicoat^{®} IR is a hydrophilic powder readily soluble in water, white to light yellow. To improve its flow, approximately 0.3% of colloidal silica is added. In a special embodiment of the invention graft copolymer of polyvinyl alcohol with polyethylene glycol can be used in a form of solid solution with polyvinyl alcohol, which is sold under trade name Kollicoat Protect and which is composed of 55-65w/w% of graft copolymer of polyvinyl alcohol with polyethylene glycol, 35-45w/w% of polyvinyl alcohol and 0.1 to 0.3w/w% silicon dioxide.

Furthermore, lubricants may also be present as excipients, such as stearic acid, magnesium stearate, calcium stearate, sodium laurylsulphate, hydrogenated vegetable oil, hydrogenated castor oil, sodium stearyl fumarate, talc, macrogols, or any mixtures thereof.

The compositions according to the present invention can comprise also from diluents/fillers, namely microcrystalline cellulose, powdered cellulose, lactose (preferably anhydrous or monohydrate), compressible sugars, fructose, dextrates, other sugars and/or theirs derivatives such as mannitol, sorbitol, maltitol, xylitol, lactitol, saccharose, raffinose, trehalose, fructose or mixture thereof, siliconised microcrystalline cellulose, calcium hydrogen phosphate (anhydrous or hydrated), calcium carbonate, calcium lactate,coprocessed multifunctional excipients such as fructose and starch (commercially available as Advantose FS 95), microcrystalline cellulose and guar gum (commercially available as Avicel CE15), lactose and cellulose (commercially available as Cellactose and MicroceLac), F-Melt, calcium carbonate and sorbitol (commercially available as Formaxx), mannitol, superfine crospvidone (commercially available as Kollidon CL-SF) and polyvinyl acetate dispersion stabilized with povidone and sodium lauryl sulfate (commercially available as Kollicoat SR 30 D) (the multifunctional excipient is commercially available as Ludiflash), lactose, povidone (commercially available as Kollidon 30) and crospovidone (commercially available as Kollidon CL) (the multifunctional excipient is commercially available as Ludipress), microcrystalline cellulose, hypromellose and crospovidone (commercially available as PanExcea MCC333G), anhydrous lactose and lactitiol (commercially available as Pharmatose DCL40), microcrystalline cellulose and colloidal silicon dioxide (commercially available as Prosolv), corn starch and pregelatinized starch (commercially available as StarCap 1500), lactose and maize starch (commercially available as Star lac), xylitol and polydextrose (commercially available as Xylitab 100), xylitol and sodium carboxymethylcellulose (commercially available as Xylitab), sucrose and dextrin (commercially available as Di-Pac), microcrystalline cellulose and calcium phosphate (commercially available as Celocal), fructose and starch (commercially available as Advantose FS95), maltose (commercially available as Advantose 100), calcium carbonate and starch (commercially available as Barcroft CS90), Al₂(OH)₃, Mg(OH)₂ and sorbitol (commercially available as Barcroft Premix St), vinyl acetate and vinyl pyrrolidone (commercially available as Plasdone S-630 copovidone), calcium carbonate and acacia (commercially available as Carbofarma GA10), calcium carbonate and maltodextrin (commercially available as Carbofarma GM11) or mixtures thereof. For the diluents used it is advantageous to select those having good compressibility and flowability such as those designed for direct compression and can be preferably selected from coprocessed excipients wherein two or more excipients are combined into coprocessed single particles and/or processed single expients such as spray dryed or granulated excipient. Particle size of diluents used is important for processibility of the composition. Preferably diluents used extragranularly have the average particle size in the range 50 to 500 µm, preferably 75 to 400 µm. Average particle size of diluents used intragranularly is preferably in the range 20 to 300 µm and even more preferably 30 to 150 µm.

The compositions of the present inventions may include also surfactants. Surfactant molecules may be classified based on nature of the hydrophilic group within the molecule. The four main groups of surfactants are defined as follows:
1. *Anionic surfactants,* where the hydrophilic group carries a negative charge, such as carbonyl (RCOO⁻), sulphonate (RSO3⁻) or sulphate (ROSO3⁻). Examples of pharmaceutical importance include potassium laurate, CH₃(CH₂)₁₀COO⁻K⁺, and sodium lauryl sulphate, CH₃(CH₂)₁₁SO₄⁻Na⁺.
2. *Cationic surfactants,* where the hydrophilic group carries a positive charge (e.g., quaternary ammonium halides, R₄N⁺Cl⁻). Examples of pharmaceutical importance include cetrimide, a mixture consisting mainly of tetradecyl (ca. 68%), dodecyl (ca. 22%), and hexadecyltrimethylammonium bromides (ca. 7%), as well as benzalkonium chloride, a mixture of alkylbenzyldimethylammonium chlorides of the general formula [C₆H₅CH₂N⁺(CH₃)₂R]Cl⁻, where R represents a mixture of the alkyls from C₈H₁₇ to C₁₈H₃₇.
3. *Ampholytic surfactants (also called zwitterionic surfactants)*, where molecule contains, or can potentially contain, both a negative and a positive charge, (e.g., the sulfobetaines, RN⁺(CH₃)₂CH₂CH₂SO₃⁻). Examples of pharmaceutical importance include *N*-Dodecyl-*N,N*-Dimethylbetaine, C₁₂H₂₅N⁺(CH₃)₂CH₂COO⁻.
*4. Nonionic surfactants,* where the hydrophile carries no charge but derives its water solubility from highly polar groups such as hydroxyl or polyoxyethylene
(OCH₂CH₂O-) groups. Examples of pharmaceutical importance include polyoxyethylated glycol monoethers (e.g. cetomacrogol), sorbitan esters (Spans^{®}) and polysorbates (Tweens^{®}).

A special example of surfactants are poloxamers, that are defined as nonionic polyoxyethylene-polyoxypropylene copolymers. They are chemically similar in composition, differing only in the relative amounts of propylene and ethylene oxides added during manufacture.

Lubricants, glidants and antiadherents if used in solid pharmaceutical compositions of the present invention, are herein not defined as surface-acting agents (surfactants).

Excipients, which may be present, may have multiple functions, such as one excipient may be diluent and additionally binder and disintegrant such as microcrystalline cellulose and/or starch and/or its derivatives, which can have a function of diluent/filler and binder. Also any other excipient described in the present invention may have multiple functions. However, individual functions of the multifunctional excipients could not be quantitatively evaluated.

Optionally, tablet cores can be coated with conventional materials used for film coating, i.e. as described in G. Cole (ed.), Pharmaceutical Coating Technology (1995). Film coating formulations usually contain the following components:
- at least one polymer,
- at least one plasticizer, at least one colorant and/or opacifier, and
- at least one vehicle (solvent).

In film coating suspension we can use minor quantities of flavours, surfactants and waxes. The vast majority of the polymers used in film coating are either cellulose derivatives, such as the cellulose ethers, or acrylic polymers and co-polymers. Occasionally encountered are high molecular weight polyethylene glycols, polyvinyl pyrrolidone, polyvinyl alchohol and waxy materials. Typical cellulose ethers are hydroxyethylcellulose, hydroxypropylcellulose, hydroxypropylmethylcellulose, methylcellulose and the like. Acrylic polymers comprise a group of synthetic polymers with diverse functionalities.

In a special embodiment of the invention film coating applied onto the tablet cores can have special functions such as decreased permeability for oxygen and/or moisture, decreased solubility in physiological conditions such as gastro-resistant coatings and/or prolonged release coatings. Functional film coatings are based on special properties of polymers used in the film coating dispersions. Oxygen and/or moisture decreased permeability can be achieved by film coating based on polymers such as polyvinyl alcohol, hydropropyl cellulose, block copolymer of polyethylene glycol and polyvinyl alcohol (commercially available as Kollicoat IR or Kollicoat Protect), aminoalkyl methacrylate copolymers (commercially available as Eudragit EPO).

Gastro-resistance of coated tablets can be achieved by using polymers insoluble in acidic media with pH below 4, preferably below 5 and even more preferably below 5.5 such as methacrylic acid copolymers (commercially available as Eudragit L and/or Eudragit S types), hydroxypropyl methyl cellulose acetatesuccinate, cellulose acetate phthalate, hydroxypropyl methyl cellulose phthalate.

Prolonged release coatings are based on polymers insoluble in water such as ethyl cellulose, methacrylic ester copolymers (commercially available as Eudragit NE and/or Eudragit NME), ammonio methacrylate copolymers (commercially available as Eudragit RS and/or Eudragit RL), polyvinyl acetate (commercially available as Kollicoat SR).

Composition of some film coatings of tablet cores can be further modified to enhance swelling and permeability by the incorporation of materials such as water soluble cellulose ethers and starches in order to ensure complete disintegration /dissolution of the film.

The commonly used plasticizers can be categorized into three groups: polyols (glycerol, propylene glycol, polyethylene glycols with molecular weight in the range 200 to 10,000, preferably 300 to 8,000), organic esters (phthalate esters, dibutyl sebacetate, citrate esters such as triethyl citrate, triacetin), and oils/glycerides (castor oil, acetylated monoglycerides, fractionated coconut oil).

Colourants/opacifiers are classified into several groups: organic dyes and their lakes, inorganic colours, natural colours.

Different materials from each group can be combined in defined ratios. Film coating suspensions can be used as ready-to-make preparations that are available on the market, e.g. as Opadry^{®} of different types
Film coating dispersion can be prepared by using different solvents (water, alcohols, ketones, esters, chlorinated hydrocarbons, preferably water.

A composition of coating suspension (calculated on dry material) is particularly preferred which comprises:
1-99% by weight of polymer, preferably 1-95% of polymer,
1-50% by weight of plasticizer calculated on the amount of polymer used in the coating, preferably 5-40% of plasticizer,
0.1-20% of colourant/opacifier, preferably 0.1-10% of colourant/opacifier.

The immediate release dosage form may also include a material that improves the processing of the release controlling agents. Such materials are generally referred as plasticizers. Preferred plasticizers include acetylated monoglycerides, butyl phthalyl butyl glycolate, dibutyl tartrate, diethyl phthalate, dimethyl phthalate, ethyl phthalyl ethyl glycolate, glycerin, ethylene glycol, propylene glycol, triethyl citrate, triacetin, tripropinoin, diacetin, dibutyl phthalate, acetyl monoglyceride, polyethylene glycols, castor oil, polyhydric alcohols, acetate esters, glycerol triacetate, acetyl triethyl citrate, dibenzyl phthalate, dihexyl phthalate, butyl octyl phthalate, diisononyl phthalate, butyl octyl phthalate, dioctyl azelate, epoxidised tallate, triisoctyl trimellitatae, diethylhexyl phthalate, di-n-tridecyl phthalate, tri-2-ethylhexyl trimellitatate, di-2-ethylhexyl adipate, di-ethylhexyl sebacate, di-2-ethylhexyl azelate, dibutyl sebacate, glyceryl monocaprylate, glycerol distearate and glyceryl monocaprate.

Mixing of the excipients with the active ingredient may be effected in a conventional device used for mixing of powders, e.g. a motionless (passive) mixer, fluidized bed mixer, diffusion mixer, biconic diffusion mixer, uniconic mixer, biconic mixer, turbular mixer, cubic mixer, planetary mixer, Y-shaped mixer, V-shaped mixer or high-shear mixer.

In the process of the invention as described above, the compression, in particular to cores/tablets, can be effected using an automatic rotary compressing machine from different manufacturers of equipment for use in pharmaceutical industry.

Conventional equipment for applying a film coating, such as fluid bed (e.g. fluid bed coating system) or conventional coating pans for use in pharmaceutical industry.

One or more of these additives can be selected and used by the skilled artisan having regard to the particular desired properties of the solid oral dosage form by routine experimentation and without any undue burden. Furthermore, the composition of the present invention comprises the active ingredient solifenacin succinate and at least one pharmaceutically acceptable excipient.

The amount of each type of additive employed, e.g. glidant, binder, disintegrant and/or superdisintegrant, binder, lubricant, glidant, filler and optionally film coating layer may vary within ranges conventional in the art.

According to embodiments of the invention:
- The amount of disintegrant and/or superdisintegrant may vary in the range 1 to 90%, preferably 1-40% by weight, more preferably 1-25% by weight, each based on the total weight of the solid oral dosage form.
- The amount of binder may vary in the amount 1 to 90%, preferably 1-50% by weight, each based on the total weight of the solid oral dosage form.
- The amount of lubricant may vary in the amount 0.1 to 10% by weight, each based on the total weight of the solid oral dosage form.
- The amount of filler may vary within a range 20-99%, preferably
50-99% by weight, each based on the total weight of the solid oral dosage form.

Optionally, the amount of film coating layer may vary from 0.1 to 10% by weight, each based on the total weight of the solid oral dosage form. The thickness of the coating is dependent on its function and can be typically in the range 1 to 140 µm, preferably 2 to 120 µm.

The total sum of the combination of components of the formulations of the present invention is 100%. Preferably, the pharmaceutical composition according to the present invention comprises pharmaceutically acceptable excipients selected from a group as described above.

Described is that in an embodiment the composition comprises active ingredient and at least one pharmaceutically acceptable excipient as listed above.

The pre-mixes as well as the pharmaceutical composition and the final solid oral dosage form may be embedded in a gaseous mixture, wherein the oxygen may be present at a concentration of between approximately 1 % to 16 % (v/v), preferably at a concentration of between approximately 2 to 12 % (v/v), most preferably at a concentration of between approximately 4 to 10 % (v/v). Preferably nitrogen or argon can be used as inert gas atmosphere in the packaging procedure, wherein nitrogen is especially preferred.

The pharmaceutical composition of the present invention is packed into primary packaging material under ambient conditions.

Optionally, if the active compounds of the present composition and/ or the composition itself are exhibited to a reduced oxygen partial pressure and/or inert atmosphere such nitrogen, the formulation is preferably enclosed in a substantially gas exchange non-permeable material and an atmosphere with reduced oxygen partial pressure is contained in the packaging. The substantially gas exchange non-permeable package is preferably selected from the group consisting of an Al/Al blister, an Al-polychloro-3-fluoroethylene homopolymer/PVC laminate blister, Aclar^{®} blister, or a bottle.

The formulations of the invention are preferably in form of a coated or uncoated tablet, such as fast disintegrating tablet or orally disintegrating tablet, a capsule or in form of pellets. The composition can also take the form of a powder mixture, a granulate or mini tablets filled in capsules. An immediate release composition is preferable. An immediate release composition, from which at least 70 weight % of incorporated active agent is released in 45 minutes is preferable.

The absolute amounts of each additive and the amounts relative to other additives is similarly dependent on the desired properties of the solid oral dosage form and may also be chosen by the skilled artisan by routine experimentation without undue burden. For example, the solid oral dosage form may be chosen to exhibit accelerated and/or delayed release of the active agent with or without quantitative control of the release of active agent.

Thus, where accelerated release is desired, e.g. about 90% release within a ten minute, more particularly a five minute period, a disintegrant and/or superdisintegrant or reactive additives (effervescent mixtures) that effect rapid disintegration of the tablet in the presence of water, can be used, for example so-called effervescent tablets that contain an acid in solid form, typically citric acid, which acts in water on a base containing chemically combined carbon dioxide, for example sodium hydrogen carbonate or sodium carbonate, and releases carbon dioxide.

Where prolonged release is desired one can employ pellet and/or tablet coating technology, wax matrix systems, polymer matrix tablets or polymer coatings conventional in the art.

In case delayed release of active agent is desired, the drug containing core such as pellet, granule or tablet is coated with a film coating based on polymers insoluble in acidic media having pH below 4.5, preferably 5.0 and most preferably 5.5.

Whereas prolonged release is desired one can employ pellet coating technology, wax matrix systems, polymer matrix tablets or polymer coatings conventional in the art.

Quantitative control of the release of the active agent can be achieved by conventional techniques known in the art. Such dosage forms are known as oral osmotic systems (OROS), coated tablets, matrix tablets, press-coated tablets, multilayer tablets and the like.

In order to achieve good bioavailability and solve the problems due to the strong aggregation properties of solifenacin and its salts leading to poor content uniformity and sticking of the mixture to the punches during compression, preferably pharmaceutical excipients are used in which, cumulatively, 10% of particles of the main excipient, preferably a filler/ diluent, have a diameter larger than 140 µm, preferably, larger than 160 µm, most preferably larger than 160 µm, measured by a pre-specified analytical method, e.g. using a Malvern 2000 Scirocco dispersion unit, in particular according to the specifications of the manufacturer.

In another embodiment of the present invention, it is necessary to use pharmaceutical excipients in which, cumulatively, 50% of particles of the main excipient, preferably a filler/ diluent, have a diameter larger than 55 µm, preferably, larger than 60 µm, more preferably larger than 70 µm, most preferably larger than 80 µm, measured by a pre-specified analytical method, e.g. using a Malvern 2000 Scirocco dispersion unit.

In yet another embodiment of the present invention, pharmaceutical excipients are used in which, cumulatively, the average particle size (diameter of the particle) of the main excipient, preferably a filler/ diluent, is larger than 60 µm, preferably, larger than 70 µm, more preferably larger than 80 µm, most preferably larger than 90 µm, measured by a pre-specified analytical method, e.g. using a Malvern 2000 Scirocco dispersion unit.

In still yet another embodiment of the present invention, pharmaceutical excipients are used in which less than 10% of particles has a diameter less than 32 µm, 20-45% of particles has a diameter less than 100 um and more than 80% of particles has a diameter less than 200 µm, measured by a pre-specified method, e.g. using air-jet sieve analysis.

In still yet another embodiment of the present invention, pharmaceutical excipients are used in which less than 20% of particles has a diameter less than 63 µm, 40-75% of particles has a diameter less than 180 µm, more than 85% of particles has a diameter less than 400 µm and more than 97% of particles has a diameter less than 630 µm, measured by a pre-specified analytical method, e.g. air-jet sieve analysis.

In another embodiment of the present invention, pharmaceutical excipients are used in which less than 25% of particles has a diameter less than 63 µm, 60-90% of particles has a diameter less than 250 µm and more than 96% of particles has a diameter less than 500 µm, measured by a pre-specified method, e.g. using air-jet sieve analysis.

In yet another embodiment of the present invention, pharmaceutical excipients are used in which less than 15% of particles has a diameter less than 45 µm, 30-60% of particles has a diameter less than 100 µm and more than 98% of particles has a diameter less than 250 µm, measured by a pre-specified method, e.g. using air-jet sieve analysis.

Physical characteristics of the tabletting mixture mainly depends on physical characteristics of the active ingredient and excipients. Excipients, having a flow rate over the limit of 40 seconds result in a non-homogeneous tabletting mixture which further results in poor content uniformity of the active ingredient in the final product. It is preferable to use pharmaceutical excipients in which the flow rate of the main excipient, preferably a filler/ diluent, is below 40 seconds, preferably below 35 seconds, most preferably below 30 seconds, measured by a pre-specified analytical method, e.g. determined on Pharmatest PTG.

In another embodiment of the present invention, the angle of repose of the tabletting mixture is a parameter, which determines flow characteristics. It has been found out that the excipients, having an angle of repose over the limit of 36°, result in non-homogeneous tabletting mixture which results in poor content uniformity of the active ingredient in the final product. It is necessary to use pharmaceutical excipients in which the angle of repose of the main excipient, preferably a filler, is below 36°, preferably below 34°, most preferably below 32°, measured by a pre-specified analytical method, e.g. determined on Pharmatest PTG.

In another embodiment of the present invention, the Hausner ratio of the tabletting mixture is a parameter, which determines compressing properties. Values for Hausner ratio varies from 1.2 for free flowing powders to 1.6 for cohesive powders as described in Gibson M. (ed.), Pharmaceutical preformulation and formulation, A practical form drug candidate selection to commercial dosage form (2004). The excipients, having a Hausner ratio over 1.4, may result in non-homogeneous tabletting mixture which could result in poor content uniformity of the active ingredient in the final product. It is necessary to use pharmaceutical excipients in which the Hausner ratio of the main excipient, preferably a filler, is below 1.40, preferably below 1.30, more preferably below 1.28, most preferably below 1.26.

By "effective amount" is meant the amount of active agent which halts or reduces the progress of the condition being treated or which otherwise completely or partly cures or acts palliatively on the condition. Such an amount can be easily determined by a person skilled in the art by routine experimentation and with no undue burden.

The formulations of the invention are preferably provided comprising an amount of solifenacin in an amount of 1-10 mg, preferably in the amount of 3.8 mg and 7.5 mg of solifenacin, solifenacin in the formulation of the present invention being in the form of solifenacin succinate. Preferably, solifenacin in the formulation of the present invention is in the form of solifenacin succinate, wherein the amount of solifenacin succinate in the formulation of the present invention is 5 mg and 10 mg. Most preferably, crystalline solifenacin succinate is applied.

The terms "active pharmaceutical ingredient" and "active ingredient" refer to solifenacin salts, which is solifenacin succinate. The crystalline form is used.

In yet another preferred embodiment of the formulation of the present invention, the average particle size of solifenacin succinate is less than 600 µm, more preferably less than 500 µm, even more preferably less than 250 µm, more preferably less than 200 µm, even more preferably less than 150 µm and most preferably less than 100 µm, as measured by laser light diffraction. The maximum primary particle diameter is preferably 2000 µm, more preferably 1000 µm as determined by microscopic analysis.

The term "average particle size" as used herein refers to the volume mean particle diameter. The term "maximum primary particle diameter" as used herein refers to a diameter measured as the longest distance of a singular particle.

In the context of the present invention, the term "main excipient" can have in particular the meaning of the or an excipient being present in the highest amount of weight in a solid dosage form, when comparing the amounts of weight of all pharmaceutical excipients present in the solid oral dosage form.

Methods for determining the solvent content, in particular the water content, of the ingredients used for producing a solid oral dosage form and/or the intermediate products thereof, and/or the solvent content, in particular the water content, of the intermediate products, in particular mixture of ingredients, granulate and/or comprimate, and/or the solvent content, in particular the water content, of the solid oral dosage form according to one embodiment of the invention are known to a person skilled in the art. According to one embodiment thermogravimetry is employed, for example using a Mettler Toledo TGA/DSC 1 according to the manual instructions provided for Mettler Toledo TGA/DSC 1 (Mettler-Toledo GmbH, Giessen, DE). According to the general knowledge in the art, the analytic device(s) used, in particular a thermogravimetric analysis (TGA) device, may be coupled with further analytic units, for example for analyzing evolving gases, in particular with a mass spectrometer (MS) or with infrared spectroscopy (1R) devices.

According to a further aspect, the present invention provides solid oral dosage forms and/or mixtures and/or comprimates and/or granulates obtainable according to a process of the present invention or obtainable according to one or more steps thereof.

According to yet another aspect, the present invention provides the use of solid oral dosage forms and/or comprimates and/or granulates obtainable according to a process of the present invention or one or more step(s) thereof for the preparation of a medicament for the treatment of, alleviation and/or prevention of overactive bladder syndrome, urinary urgency and increased urinary frequency.

According to yet another aspect, the solid oral dosage form of the present invention is for use in the treatment of, alleviation and/or prevention of overactive bladder syndrome, urinary urgency and increased urinary frequency.

If not indicated otherwise, all percentages given herein are wt. %, based on the total weight of the formulation. Preferably, the present formulation comprises solifenacin succinate in an amount of 0.1-20%, more preferably 0.5-15%, even more preferably 1-15% and most preferably 1-10%.

Described herein is:
1.) A process for the preparation of a solid oral dosage form comprising:
   a.) an effective amount of crystalline solifenacin or its pharmaceutically acceptable salt,
   b.) pharmaceutically acceptable additives suitable for the preparation of solid oral dosage forms,
   in the absence of water, preferably in the absence of a solvent.
2.) A process for the preparation of solid dosage forms according to item 1, comprising the steps of:
   i) optionally grinding crystalline solifenacin or its pharmaceutically active salts and pharmaceutically acceptable excipients,
   ii) subjecting a homogenous mixture of the ground active ingredient and excipients to compression to form a comprimate,
   iii) converting the comprimate to form a granulate and
   iv) compressing/ tabletting the granulate to form the solid oral dosage form.
3.) A process according to items 1 and 2, characterized in that it is a roller compaction method.
4.) A process for the preparation of solid dosage forms according to item 1, comprising the steps of:
   a.) homogenously mixing crystalline solifenacin or its pharmaceutically acceptable salt with pharmaceutically acceptable excipients,
   a.) compressing/ tabletting of the mixed active ingredient and pharmaceutically acceptable excipients to form a solid dosage form.
5.) A process for the preparation of solid dosage forms according to items 1 to 4, characterized in that crystalline solifenacin succinate is used.
6.) A process for the preparation of solid dosage forms according to items 1 to 4, characterized in that crystalline solifenacin hydrogen sulphate (VI) is used.
7.) A process for the preparation of solid dosage forms according to items 1 to 6, characterized in that pharmaceutical excipients are used in which, cumulatively, the average particle size of the main excipient, preferably a filler or diluent, is larger than 60 µm, preferably, larger than 70 µm, more preferably larger than 80 µm, most preferably larger than 90 µm, measured by a pre-specified analytical method, e.g. using a Malvern 2000 Scirocco dispersion unit.
8.) A process for the preparation of solid dosage forms according to items 1 to 7, characterized in that pharmaceutical excipients are used in which the flow rate of the main excipient, preferably a filler or diluent, is below 40 seconds, preferably below 35 seconds, most preferably below 30 seconds, measured by a pre-specified analytical method, e.g. determined on Pharmatest PTG.
9.) A process for the preparation of solid dosage forms according to items 1 to 8, characterized in that pharmaceutical excipients are used in which the angle of repose of the main excipient, preferably a filler or diluent, is below 36°, preferably below 34°, most preferably below 32°, measured by a pre-specified analytical method, e.g. determined on Pharmatest PTG.
10.) A process for the preparation of solid dosage forms according to items 1 to 9, characterized in that pharmaceutical excipients are used in which the Hausner ratio of the main excipient, preferably a filler or diluent, is below 1.40, preferably below 1.30, more preferably below 1.28, most preferably below 1.26.
11.) A solid oral dosage form prepared by a process in the absence of water, preferably in the absence of a solvent, comprising one or more disintegrants and/or superdisintegrants in the range 1 to 90%, preferably 1-40% by weight, more preferably 1-25% by weight, a binder in the amount 1 to 90%, preferably 1-50% by weight, a lubricant in the amount 0.1 to 10% by weight, and a filler or diluent within a range 1 to 99%, preferably 20-99%, more preferably 50-99% by weight, so that the total sum of the combination of components of the formulation is 100%.
12.) A solid oral dosage form according to item 11, characterized in that it is prepared by direct compression or roller compaction.
13.) A solid oral dosage form according to items 11 or 12, characterized in that average particle size of the main excipient, preferably a filler or diluent, is larger than 60 µm, preferably, larger than 70 µm, more preferably larger than 80 µm, most preferably larger than 90 µm, measured by a pre-specified analytical method, e.g. using a Malvern 2000 Scirocco dispersion unit.
14.) A solid oral dosage form according to items 11 to 13, characterized in that the flow rate of the main excipient, preferably a filler or diluent, is below 40 seconds, preferably below 35 seconds, most preferably below 30 seconds, measured by a pre-specified analytical method, e.g. determined on Pharmatest PTG.
15.) A solid oral dosage form according to items 11 to 14, characterized in that the main excipient is lactose monohydrate, microcrystalline cellulose or mannitol.
16.) Solid oral dosage form according to any of items 11 to 15, for use in the treatment of, alleviation and/or prevention of overactive bladder syndrome, urinary urgency and increased urinary frequency.

The invention is illustrated by the following examples which in no way restrict its scope.

### Examples

### Example 1

20 g of solifenacin succinate, 205 g of lactose monohydrate (particles A) (table 1), 60 g of maize starch and 12 g of hydroxypropylmethylcellulose were screened through the sieve with sieve openings 0.6 mm and homogeneously mixed.

**Table 1A. Physical characteristics of lactose monohydrate (particles A).**

| Analysis | Result |
|---|---|
| Sieving analysis (determined on Alpina 200 LS): | |
| - particles passed the sieve with sieve openings 71 µm | 6.7% |
| - particles passed the sieve with sieve openings 125 µm | 34.9% |
| - particles passed the sieve with sieve openings 250 µm | 90.6% |
| - particles passed the sieve with sieve openings 500 µm | 100% |
| Flow rate (determined on Pharmatest PTG) | 16.0 s |
| Angle of repose (determined on Pharmatest PTG) | 31.8° |
| Bulk volume (determined on Engelsmann) | 1.92 mL/g |
| Tapped volume (determined on Engelsmann) | 1.56 mL/g |
| Hausner index | 1.23 |
| Particle size distribution (determined using a Malvern 2000 Scirocco dispersion unit): | |
| - average particle size | 95 µm |
| - 10% of particles smaller than | 17 µm |
| - 10% of particles larger than | 191 µm |
| - 50% of particles larger than | 81 µm |

Finally, 3 g of magnesium stearate was added to prepare the tabletting mixture. The physical characteristics of the tabletting mixture are presented in table 1B'.

**Table 1B. Physical characteristics of tabletting mixture.**

| Analysis | Result |
|---|---|
| Sieving analysis (performed on Alpina 200 LS): | |
| - particles passed the sieve with sieve openings 71 µm | 29% |
| - particles passed the sieve with sieve openings 125 µm | 46.3% |
| - particles passed the sieve with sieve openings 250 µm | 89.5% |
| - particles passed the sieve with sieve openings 500 µm | 100% |
| Flow rate (determined on Pharmatest PTG) | 28.6 s |
| Angle of repose (determined on Pharmatest PTG) | 39.1° |
| Bulk volume (determined on Engelsmann) | 1.40 mL/g |
| Tapped volume (determined on Engelsmann) | 1.20 mL/g |
| Hausner index | 1.17 |

The tabletting mixture was compressed on an automatic rotary compressing machine Pressima into round-shaped cores with theoretical weight of 150 mg. The hardness of the cores (performed on Erweka) was 29-38 N (average of n = 20 cores was 33 N) and disintegration time (purified water, 37°C) 3-3.5 minutes.

Part of the cores were coated in automatic coating machine Manesty XL with water-based film coating suspension, containing hydroxypropylmethylcellulose (62% by weight), macrogol 6000 (11% by weight), talc (19% by weight), titanium dioxide (8% by weight) and iron oxide (<0.05% by weight, not included in total sum of the dry costituents of the film coating suspension). The theoretical weight of the film coated tablets was 154 mg.

The rest of the cores were coated in automatic coating machine Manesty XL with ready-to-use dry powder for film coating suspension, commercially available as Opadry AMB, comprising polyvinyl alcohol-partially hydrolyzed, titanium dioxide, talc, lecithin (soya) and xanthan gum. The dry powder is dispersed in purified water to prepare up to 20% w/w dispersion. The theoretical weight of the film coated tablets was 154 mg.

### Example 2 (Reference)

20 g of solifenacin succinate, 205 g of lactose monohydrate (particles B) (table 2), 60 g of maize starch and 12 g of hydroxypropylmethylcellulose were screened through the sieve with sieve openings 0.6 mm and homogeneously mixed.

**Table 2A. Physical characteristics of lactose monohydrate (particles B).**

| Analysis | Result |
|---|---|
| Sieving analysis (determined on Alpina 200 LS): | |
| - particles passed the sieve with sieve openings 71 µm | 100% |
| Flow rate (determined on Pharmatest PTG) | / |
| Angle of repose (determined on Pharmatest PTG) | / |
| Bulk volume (determined on Engelsmann) | 1.92 mL/g |
| Tapped volume (determined on Engelsmann) | 1.20 mL/g |
| Hausner index | 1.6 |
| Particle size distribution (determined using a Malvern 2000 Scirocco dispersion unit): | |
| - average particle size | 46 µm |
| - 10% of particles smaller than | 4 µm |
| - 10% of particles larger than | 114 µm |
| - 50% of particles larger than | 31 µm |
| / the measurement could not be performed due to poor flowability | |

Finally, 3 g of magnesium stearate was added to prepare tabletting mixture. The physical characteristics of the tabletting mixture are presented in table 2B'.

**Table 2B'. Physical characteristics of tabletting mixture.**

| Analysis | Result |
|---|---|
| Sieving analysis (performed on Alpina 200 LS): | |
| - particles passed the sieve with sieve openings 71 µm | 92.1% |
| - particles passed the sieve with sieve openings 125 µm | 100% |
| Flow rate (determined on Pharmatest PTG) | 29.5 s |
| Angle of repose (determined on Pharmatest PTG) | 38.5° |
| Bulk volume (determined on Engelsmann) | 1.58 mL/g |
| Tapped volume (determined on Engelsmann) | 1.20 mL/g |
| Hausner index | 1.32 |

The tabletting mixture was compressed on automatic rotary compressing machine Pressima into round-shaped cores with theoretical weight of 150 mg. The hardness of the cores (performed on Erweka) was 33-84 N (average of n = 20 cores was 57 N) and disintegration time (purified water, 37°C) 3-3.5 minutes.

Cores were coated according to a process as described in Example 1.

### Example 3

20 g of solifenacin succinate, 205 g of microcrystalline cellulose (particles A) (table 3), 60 g of maize starch and 12 g of hydroxypropylmethylcellulose were screened through the sieve with sieve openings 0.6 mm and homogeneously mixed.

**Table 3A. Physical characteristics of microcrystalline cellulose (particles A).**

| Analysis | Result |
|---|---|
| Sieving analysis (determined on Alpina 200 LS): | |
| - particles passed the sieve with sieve openings 71 µm | 18.1% |
| - particles passed the sieve with sieve openings 125 µm | 38.5% |
| - particles passed the sieve with sieve openings 250 µm | 88.6% |
| - particles passed the sieve with sieve openings 500 µm | 100% |
| Flow rate (determined on Pharmatest PTG) | 36.3 s |
| Angle of repose (determined on Pharmatest PTG) | 29.6° |
| Bulk volume (determined on Engelsmann) | 2.68 mL/g |
| Tapped volume (determined on Engelsmann) | 2.12 mL/g |
| Hausner index | 1.39 |
| Particle size distribution (determined using a Malvern 2000 Scirocco dispersion unit): | |
| - average particle size | 234 µm |
| - 10% of particles smaller than | 87 µm |
| - 10% of particles larger than | 403 µm |
| - 50% of particles larger than | 219 µm |

Finally, 3 g of magnesium stearate was added to prepare tabletting mixture. The physical characteristics of the tabletting mixture are presented in table 3B'.

**Table 3B'. Physical characteristics of tabletting mixture.**

| Analysis | Result |
|---|---|
| Sieving analysis (performed on Alpina 200 LS): | |
| - particles passed the sieve with sieve openings 71 µm | 34.3% |
| - particles passed the sieve with sieve openings 125 µm | 47.3% |
| - particles passed the sieve with sieve openings 250 µm | 84.1% |
| - particles passed the sieve with sieve openings 500 µm | 100% |
| Flow rate (determined on Pharmatest PTG) | 38.4 s |
| Angle of repose (determined on Pharmatest PTG) | 42.2° |
| Bulk volume (determined on Engelsmann) | 2.04 mL/g |
| Tapped volume (determined on Engelsmann) | 1.62 mL/g |
| Hausner index | 1.26 |

The tabletting mixture was compressed on automatic rotary compressing machine Pressima into round-shaped cores with theoretical weight of 150 mg. The hardness of the cores (performed on Erweka) was 56-74 N (average of n = 20 cores was 64 N) and disintegration time (purified water, 37°C) 20-30 seconds.

Cores were coated according to a process as described in Example 1.

### Example 4 (Reference)

20 g of solifenacin succinate, 205 g of microcrystalline cellulose (particles B) (table 4), 60 g of maize starch and 12 g of hydroxypropylmethylcellulose were screened through the sieve with sieve openings 0.6 mm and homogeneously mixed.

**Table 4A. Physical characteristics of microcrystalline cellulose (particles B).**

| Analysis | Result |
|---|---|
| Sieving analysis (determined on Alpina 200 LS): | |
| - particles passed the sieve with sieve openings 71 µm | 81.3% |
| - particles passed the sieve with sieve openings 125 µm | 99.5% |
| - particles passed the sieve with sieve openings 250 µm | 100% |
| Flow rate (determined on Pharmatest PTG) | 119.0 s |
| Angle of repose (determined on Pharmatest PTG) | 36.1° |
| Bulk volume (determined on Engelsmann) | 3.00 mL/g |
| Tapped volume (determined on Engelsmann) | 2.32 mL/g |
| Hausner index | 1.39 |
| Particle size distribution (determined using a Malvern 2000 Scirocco dispersion unit): | |
| - average particle size | 59 µm |
| - 10% of particles smaller than | 19 µm |
| - 10% of particles larger than | 110 µm |
| - 50% of particles larger than | 53 µm |

Finally, 3 g of magnesium stearate was added to prepare tabletting mixture. The physical characteristics of the tabletting mixture are presented in table 4B'.

**Table 4B'. Physical characteristics of tabletting mixture.**

| Analysis | Result |
|---|---|
| Sieving analysis (performed on Alpina 200 LS): | |
| - particles passed the sieve with sieve openings 71 µm | 78.4% |
| - particles passed the sieve with sieve openings 125 µm | 97.5% |
| - particles passed the sieve with sieve openings 250 µm | 100% |
| Flow rate (determined on Pharmatest PTG) | 73.9 s |
| Angle of repose (determined on Pharmatest PTG) | 38.5° |
| Bulk volume (determined on Engelsmann) | 2.60 mL/g |
| Tapped volume (determined on Engelsmann) | 1.86 mL/g |
| Hausner index | 1.40 |

The tabletting mixture was compressed on automatic rotary compressing machine Pressima into round-shaped cores with theoretical weight of 150 mg. The hardness of the cores (performed on Erweka) was 48-58 N (average of n = 20 cores was 53 N) and disintegration time (purified water, 37°C) 20 seconds.

Cores were coated according to a process as described in Example 1.

### Example 5

20 g of solifenacin succinate, 205 g of mannitol (particles A) (table 5), 60 g of maize starch and 12 g of hydroxypropylmethylcellulose were screened through the sieve with sieve openings 0.6 mm and homogeneously mixed.

**Table 5A. Physical characteristics of mannitol (particles A).**

| Analysis | Result |
|---|---|
| Sieving analysis (determined on Alpina 200 LS): | |
| - particles passed the sieve with sieve openings 71 µm | 6.9% |
| - particles passed the sieve with sieve openings 125 µm | 29.1% |
| - particles passed the sieve with sieve openings 250 µm | 66.7% |
| - particles passed the sieve with sieve openings 500 µm | 96.2% |
| - particles passed the sieve with sieve openings 710 µm | 100% |
| Flow rate (determined on Pharmatest PTG) | 19.8 s |
| Angle of repose (determined on Pharmatest PTG) | 32.8° |
| Sieving analysis (determined on Alpina 200 LS): | |
| Bulk volume (determined on Engelsmann) | 1.92 mL/g |
| Tapped volume (determined on Engelsmann) | 1.56 mL/g |
| Hausner index | 1.23 |

| Particle size distribution (determined using a Malvern 2000 Scirocco dispersion unit): | |
|---|---|
| - average particle size | 134 µm |
| - 10% of particles smaller than | 20 µm |
| - 10% of particles larger than | 310 µm |
| - 50% of particles larger than | 93 µm |

Finally, 3 g of magnesium stearate was added to prepare tabletting mixture. The physical characteristics of the tabletting mixture are presented in table 5B'.

**Table 5B'. Physical characteristics of tabletting mixture.**

| Analysis | Result |
|---|---|
| Sieving analysis (performed on Alpina 200 LS): | |
| - particles passed the sieve with sieve openings 71 µm | 39.4% |
| - particles passed the sieve with sieve openings 125 µm | 60.2% |
| - particles passed the sieve with sieve openings 250 µm | 84.2% |
| - particles passed the sieve with sieve openings 500 µm | 98.9% |
| - particles passed the sieve with sieve openings 710 µm | 100% |
| Flow rate (determined on Pharmatest PTG) | 25.3 s |
| Angle of repose (determined on Pharmatest PTG) | 39.6° |
| Bulk volume (determined on Engelsmann) | 1.70 mL/g |
| Tapped volume (determined on Engelsmann) | 1.34 mL/g |
| Hausner index | 1.27 |

The tabletting mixture was compressed on automatic rotary compressing machine Pressima into round-shaped cores with theoretical weight of 150 mg. The hardness of the cores (performed on Erweka) was 52-63 N (average of n = 20 cores was 57 N) and disintegration time (purified water, 37°C) 2-2.5 minutes.

Cores were coated according to a process as described in Example 1.

### Example 6 (Reference)

20 g of solifenacin succinate, 205 g of mannitol (particles B) (table 6), 60 g of maize starch and 12 g of hydroxypropylmethylcellulose were screened through the sieve with sieve openings 0.6 mm and homogeneously mixed.

**Table 6A. Physical characteristics of mannitol (particles B).**

| Analysis | Result |
|---|---|
| Sieving analysis (determined on Alpina 200 LS): | |
| - particles passed the sieve with sieve openings 71 µm | 54.2% |
| - particles passed the sieve with sieve openings 125 µm | 97.3% |
| - particles passed the sieve with sieve openings 250 µm | 100% |
| Flow rate (determined on Pharmatest PTG) | / |
| Angle of repose (determined on Pharmatest PTG) | / |
| Bulk volume (determined on Engelsmann) | 1.80 mL/g |
| Tapped volume (determined on Engelsmann) | 1.44 mL/g |
| Hausner index | 1.25 |

| Particle size distribution (determined using a Malvern 2000 Scirocco dispersion unit): | |
|---|---|
| - average particle size | 54 µm |
| - 10% of particles smaller than | 5 µm |
| - 10% of particles larger than | 133 µm |
| - 50% of particles larger than | 32 µm |
| / the measurement could not be performed due to poor flowability | |

Finally, 3 g of magnesium stearate was added to prepare tabletting mixture. The physical characteristics of the tabletting mixture are presented in table 6B'.

**Table 6B'. Physical characteristics of tabletting mixture.**

| Analysis | Result |
|---|---|
| Sieving analysis (performed on Alpina 200 LS): | |
| - particles passed the sieve with sieve openings 71 µm | 69.5% |
| - particles passed the sieve with sieve openings 125 µm | 93.4% |
| - particles passed the sieve with sieve openings 250 µm | 100% |
| Flow rate (determined on Pharmatest PTG) | 66.2 s |
| Angle of repose (determined on Pharmatest PTG) | 36.8° |
| Bulk volume (determined on Engelsmann) | 1.54 mL/g |
| Tapped volume (determined on Engelsmann) | 1.20 mL/g |
| Hausner index | 1.28 |

The tabletting mixture was compressed on automatic rotary compressing machine Pressima into round-shaped cores with theoretical weight of 150 mg. The hardness of the cores (performed on Erweka) was 36-48 N (average of n = 20 cores was 43 N) and disintegration time (purified water, 37°C) 2 minutes.

Cores were coated according to a process as described in Example 1.

### Example 7

20 g of solifenacin succinate, 217 g of lactose monohydrate (particles A) (table 1) and 60 g of maize starch were screened through the sieve with sieve openings 0.6 mm and homogeneously mixed.

Finally, 3 g of magnesium stearate was added to prepare tabletting mixture. The physical characteristics of the tabletting mixture are presented in table 7.

**Table 7. Physical characteristics of tabletting mixture.**

| Analysis | Result |
|---|---|
| Sieving analysis (performed on Alpina 200 LS): | |
| - particles passed the sieve with sieve openings 71 µm | 24.5% |
| - particles passed the sieve with sieve openings 125 µm | 34.2% |
| - particles passed the sieve with sieve openings 250 µm | 91.6% |
| - particles passed the sieve with sieve openings 500 µm | 100% |
| Flow rate (determined on Pharmatest PTG) | 29.8 s |
| Angle of repose (determined on Pharmatest PTG) | 36.8° |
| Bulk volume (determined on Engelsmann) | 1.46 mL/g |
| Tapped volume (determined on Engelsmann) | 1.22 mL/g |
| Hausner index | 1.20 |

The tabletting mixture was compressed on automatic rotary compressing machine Pressima into round-shaped cores with theoretical weight of 150 mg. Hardness of the cores (performed on Erweka) was 31-41 N (average of n = 20 cores was 36 N) and disintegration time (purified water, 37°C) 2.5-3 minutes.

Cores were coated according to a process as described in Example 1.

### Example 8

20 g of solifenacin succinate, 205 g of lactose monohydrate (particles A) (table 1). 60 g of maize starch and 12 g of povidone were screened through the sieve with sieve openings 0.6 mm and homogeneously mixed.

Finally, 3 g of magnesium stearate was added to prepare tabletting mixture. The physical characteristics of the tabletting mixture are presented in table 8.

**Table 8. Physical characteristics of tabletting mixture.**

| Analysis | Result |
|---|---|
| Sieving analysis (performed on Alpina 200 LS): | |
| - particles passed the sieve with sieve openings 71 µm | 29.1% |
| - particles passed the sieve with sieve openings 125 µm | 48.2% |
| - particles passed the sieve with sieve openings 250 µm | 90.2% |
| - particles passed the sieve with sieve openings 500 µm | 100% |
| Flow rate (determined on Pharmatest PTG) | 20.2 s |
| Angle of repose (determined on Pharmatest PTG) | 40.4° |
| Bulk volume (determined on Engelsmann) | 1.40 mL/g |
| Tapped volume (determined on Engelsmann) | 1.20 mL/g |
| Hausner index | 1.17 |

The tabletting mixture was compressed on automatic rotary compressing machine Pressima into round-shaped cores with theoretical weight of 150 mg. The hardness of the cores (performed on Erweka) was 33-54 N (average of n = 20 cores was 43 N) and disintegration time (purified water, 37°C) 3-3.5 minutes.

Cores were coated according to a process as described in Example 1.

### Example 9

20 g of solifenacin succinate, 205 g of lactose monohydrate (particles A) (table 1), 60 g of maize starch and 12 g of Kollicoat IR were screened through the sieve with sieve openings 0.6 mm and homogeneously mixed.

Finally, 3 g of magnesium stearate was added to prepare tabletting mixture. The physical characteristics of the tabletting mixture are presented in table 9.

**Table 9. Physical characteristics of tabletting mixture.**

| Analysis | Result |
|---|---|
| Sieving analysis (performed on Alpina 200 LS): | |
| - particles passed the sieve with sieve openings 71 µm | 27.7% |
| - particles passed the sieve with sieve openings 125 µm | 47.5% |
| - particles passed the sieve with sieve openings 250 µm | 91.4% |
| - particles passed the sieve with sieve openings 500 µm | 100% |
| Flow rate (determined on Pharmatest PTG) | 26.2 s |
| Angle of repose (determined on Pharmatest PTG) | 35.3° |
| Bulk volume (determined on Engelsmann) | 1.48 mL/g |
| Tapped volume (determined on Engelsmann) | 1.22 mL/g |
| Hausner index | 1.21 |

The tabletting mixture was compressed on automatic rotary compressing machine Pressima into round-shaped cores with theoretical weight of 150 mg. The hardness of the cores (performed on Erweka) was 30-43 N (average of n = 20 cores was 36 N) and disintegration time (purified water, 37°C) 3 minutes.

Cores were coated according to a process as described in Example 1.

### Example 10

20 g of solifenacin succinate, 265 g of microcrystalline cellulose (particles A) (table 3A) and 12 g of hydroxypropylmethylcellulose were screened through the sieve with sieve openings 0.6 mm and homogeneously mixed.

Finally, 3 g of magnesium stearate was added to prepare tabletting mixture. The physical characteristics of the tabletting mixture are presented in table 10.

**Table 10. Physical characteristics of tabletting mixture.**

| Analysis | Result |
|---|---|
| Sieving analysis (performed on Alpina 200 LS): | |
| - particles passed the sieve with sieve openings 71 µm | 7.1% |
| - particles passed the sieve with sieve openings 125 µm | 27.8% |
| - particles passed the sieve with sieve openings 250 µm | 76.1% |
| - particles passed the sieve with sieve openings 500 µm | 100% |
| Flow rate (determined on Pharmatest PTG) | 36.3 s |
| Angle of repose (determined on Pharmatest PTG) | 34.2° |
| Bulk volume (determined on Engelsmann) | 2.30 mL/g |
| Tapped volume (determined on Engelsmann) | 1.94 mL/g |
| Hausner index | 1.19 |

The tabletting mixture was compressed on automatic rotary compressing machine Pressima into round-shaped cores with theoretical weight of 150 mg. The hardness of the cores (performed on Erweka) was 57-66 N (average of n = 20 cores was 61 N) and disintegration time (purified water, 37°C) 25 seconds.

Cores were coated according to a process as described in Example 1.

### Example 11

20 g of solifenacin succinate and 277 g of microcrystalline cellulose (particles A) (table 3A) were screened through the sieve with sieve openings 0.6 mm and homogeneously mixed.

Finally, 3 g of magnesium stearate was added to prepare tabletting mixture. The physical characteristics of the tabletting mixture are presented in table 11.

**Table 11. Physical characteristics of tabletting mixture.**

| Analysis | Result |
|---|---|
| Sieving analysis (performed on Alpina 200 LS): | |
| - particles passed the sieve with sieve openings 71 µm | 13.8% |
| - particles passed the sieve with sieve openings 125 µm | 22.1% |
| - particles passed the sieve with sieve openings 250 µm | 72.4% |
| - particles passed the sieve with sieve openings 500 µm | 100% |
| Flow rate (determined on Pharmatest PTG) | 32.6 s |
| Angle of repose (determined on Pharmatest PTG) | 32.9° |
| Bulk volume (determined on Engelsmann) | 2.34 mL/g |
| Tapped volume (determined on Engelsmann) | 1.96 mL/g |
| Hausner index | 1.19 |

The tabletting mixture was compressed on automatic rotary compressing machine Pressima into round-shaped cores with theoretical weight of 150 mg. The hardness of the cores (performed on Erweka) was 50-67 N (average of n = 20 cores was 57 N) and disintegration time (purified water, 37°C) 20 seconds.

Cores were coated according to a process as described in Example 1.

### Example 12

20 g of solifenacin succinate, 265 g of lactose monohydrate (particles A) (table 1A) and 12 g of hydroxypropylmethylcellulose were screened through the sieve with sieve openings 0.6 mm and homogeneously mixed.

Finally, 3 g of magnesium stearate was added to prepare tabletting mixture. The physical characteristics of the tabletting mixture are presented in table 12.

**Table 12. Physical characteristics of tabletting mixture.**

| Analysis | Result |
|---|---|
| Sieving analysis (performed on Alpina 200 LS): | |
| - particles passed the sieve with sieve openings 71 µm | 10.0% |
| - particles passed the sieve with sieve openings 125 µm | 29.7% |
| - particles passed the sieve with sieve openings 250 µm | 87.3% |
| - particles passed the sieve with sieve openings 500 µm | 100% |
| Flow rate (determined on Pharmatest PTG) | 15.4 s |
| Angle of repose (determined on Pharmatest PTG) | 31.6° |
| Bulk volume (determined on Engelsmann) | 1.64 mL/g |
| Tapped volume (determined on Engelsmann) | 1.46 mL/g |
| Hausner index | 1.12 |

The tabletting mixture was compressed on automatic rotary compressing machine Pressima into round-shaped cores with theoretical weight of 150 mg. The hardness of the cores (performed on Erweka) was 47-63 N (average of n = 20 cores was 55 N) and disintegration time (purified water, 37°C) 2.5-3 minutes.

Cores were coated according to a process as described in Example 1.

### Example 13

20 g of solifenacin succinate and 277 g of lactose monohydrate (particles A) (table 1A) were screened through the sieve with sieve openings 0.6 mm and homogeneously mixed.

Finally, 3 g of magnesium stearate was added to prepare tabletting mixture. The physical characteristics of the tabletting mixture are presented in table 13.

**Table 13. Physical characteristics of tabletting mixture.**

| Analysis | Result |
|---|---|
| Sieving analysis (performed on Alpina 200 LS): | |
| - particles passed the sieve with sieve openings 71 µm | 5.7% |
| - particles passed the sieve with sieve openings 125 µm | 23.0% |
| - particles passed the sieve with sieve openings 250 µm | 92.9% |
| - particles passed the sieve with sieve openings 500 µm | 100% |
| Flow rate (determined on Pharmatest PTG) | 13.4 s |
| Angle of repose (determined on Pharmatest PTG) | 31.8° |
| Bulk volume (determined on Engelsmann) | 1.70 mL/g |
| Tapped volume (determined on Engelsmann) | 1.48 mL/g |
| Hausner index | 1.15 |

The tabletting mixture was compressed on automatic rotary compressing machine Pressima into round-shaped cores with theoretical weight of 150 mg. The hardness of the cores (performed on Erweka) was 45-57 N (average of n = 20 cores was 50 N) and disintegration time (purified water, 37°C) 3.5-4 minutes.

Cores were coated according to a process as described in Example 1.

### Example 14

20 g of solifenacin succinate, 217 g of microcrystalline cellulose (particles A) (table 3A) and 60 g of maize starch were screened through the sieve with sieve openings 0.6 mm and homogeneously mixed.

Finally, 3 g of magnesium stearate was added to prepare tabletting mixture. The physical characteristics of the tabletting mixture are presented in table 14.

**Table 14. Physical characteristics of tabletting mixture.**

| Analysis | Result |
|---|---|
| Sieving analysis (performed on Alpina 200 LS): | |
| - particles passed the sieve with sieve openings 71 µm | 28.8% |
| - particles passed the sieve with sieve openings 125 µm | 43.3% |
| - particles passed the sieve with sieve openings 250 µm | 83.7% |
| - particles passed the sieve with sieve openings 500 µm | 100% |
| Flow rate (determined on Pharmatest PTG) | 32.1 s |
| Angle of repose (determined on Pharmatest PTG) | 42.5° |
| Bulk volume (determined on Engelsmann) | 2.00 mL/g |
| Tapped volume (determined on Engelsmann) | 1.60 mL/g |
| Hausner index | 1.25 |

The tabletting mixture was compressed on automatic rotary compressing machine Pressima into round-shaped cores with theoretical weight of 150 mg. the hardness of the cores (performed on Erweka) was 61-70 N (average of n = 20 cores was 65 N) and disintegration time (purified water, 37°C) 35 seconds.

Cores were coated according to a process as described in Example 1.

### Example 15 (Reference)

9.58 g of solifenacin hydrogen sulphate, 102.92 g of lactose monohydrate (particles A) (table 1 A), 30 g of maize starch and 6 g of hydroxypropylmethylcellulose were screened through the sieve with sieve openings 0.6 mm and homogeneously mixed.

Finally, 1.5 g of magnesium stearate was added to prepare tabletting mixture.

The tabletting mixture was compressed on automatic rotary compressing machine Pressima at the main pressure 10.2 kN into round-shaped cores with theoretical weight of 150 mg. Hardness of the cores (performed on Erweka) was 50-58 N (average of n = 20 cores was 54 N) and disintegration time (purified water, 37°C) 3 minutes.

Cores were coated according to a process as described in Example 1.

### Example 16 (Reference)

9.58 g of solifenacin hydrogensulphate and 138.92 g of lactose monohydrate (particles A) (table 1A) were screened through the sieve with sieve openings 0.6 mm and homogeneously mixed.

Finally, 1.5 g of magnesium stearate was added to prepare tabletting mixture.

The tabletting mixture was compressed on automatic rotary compressing machine Pressima at the main pressure 9.0 kN into round-shaped cores with theoretical weight of 150 mg. Hardness of the cores (performed on Erweka) was 47-62 N (average of n = 20 cores was 58 N) and disintegration time (purified water, 37°C) 6 minutes.

Cores were coated according to a process as described in Example 1.

### Example 17 (Reference)

9.58 g of solifenacin hydrogen sulphate, 108.92 g of microcrystalline cellulose (particles A) (table 3A) and 30 g of maize starch were screened through the sieve with sieve openings 0.6 mm and homogeneously mixed.

Finally, 1.50 g of magnesium stearate was added to prepare tabletting mixture.

The tabletting mixture was compressed on automatic rotary compressing machine Pressima at the main pressure 4.7 kN into round-shaped cores with theoretical weight of 150 mg. Hardness of the cores (performed on Erweka) was 56-68 N (average of n = 20 cores was 63 N) and disintegration time (purified water, 37°C) 25 seconds.

Cores were coated according to a process as described in Example 1.

### Example 18 (Reference)

9.58 g of solifenacin hydrogen sulphate, 108.92 g of mannitol (particles A) (table 5A) and 30 g of maize starch were screened through the sieve with sieve openings 0.6 mm and homogeneously mixed.

Finally, 3 g of magnesium stearate was added to prepare tabletting mixture.

The tabletting mixture was compressed on automatic rotary compressing machine Pressima at the main pressure 5.4 kN into round-shaped cores with theoretical weight of 150 mg. Hardness of the cores (performed on Erweka) was 50-61 N (average of n = 20 cores was 56 N) and disintegration time (purified water, 37°C) 2.5 minutes.

Cores were coated according to a process as described in Example 1.

### Example 19 (Reference)

9.58 g of solifenacin hydrogen sulphate, 107.42 g of lactose monohydrate (particles A) (table 1A), 30 g of dried maize starch and 1.50 g of silicon dioxide (commercially available as Syloid FP 244) were screened through the sieve with sieve openings 0.6 mm and homogeneously mixed.

Finally, 1.5 g of magnesium stearate was added to prepare tabletting mixture.

The tabletting mixture was compressed on automatic rotary compressing machine Pressima at the main pressure 10.4 kN into round-shaped cores with theoretical weight of 150 mg. Hardness of the cores (performed on Erweka) was 52-62 N (average of n = 20 cores was 56 N) and disintegration time (purified water, 37°C) 6 minutes.

Cores were coated according to a process as described in Example 1.

### Example 20 (Reference)

20 g of solifenacin succinate, 265 g of lactose monohydrate and 12 g of povidone K25 were screened through the sieve with sieve openings 0.6 mm and homogeneously mixed.

Finally, 3 g of magnesium stearate was added to prepare tabletting mixture. Tabletting mixture was compressed on automatic rotary compressing machine Pressima into round-shaped cores with theoretical weight 150 mg at main pressure 9.7 kN. Hardness of the cores (performed on Erweka) was 53-66 N (average of N=20 was 58 N) and disintegration time (purified water, 37°C) 6.5-7 minutes.

Cores were coated in automatic coating machine Manesty XL with water-based film coating suspension, containing hydroxypropylcellulose, (62% by weight), titanium dioxide (13.5% by weight), talc (18.7% by weight), triacetin (5.8% by weight) and iron oxide (<0.05% by weight, not included in total sum of the dry constituents of the film coating suspension). The theoretical weight of the film coated tablets was 154 mg.

The results of the homogeneity of the solifenacin in film coated tablets were (HPLC, average of the 2 parallel measurements for each tablet, perfoemd on 10 random selected tablets):

| | Content |
|---|---|
| 1 | 99.3% |
| 2 | 100.2% |
| 3 | 100.1% |
| 4 | 99.9% |
| 5 | 102.6% |
| 6 | 99.1% |
| 7 | 101.0% |
| 8 | 101.7% |
| 9 | 96.9% |
| 10 | 100.9% |
| average | 100.2% |
| Relative standard deviation | 1.5 |

### Example 21 (Reference)

40 g of solifenacin succinate, 524 g of lactose monohydrate and 24 g of povidone K25 were screened through the sieve with sieve openings 0.6 mm and homogeneously mixed. Finally, 3 g of magnesium stearate was added to prepare bricketting mixture. The brickettes were prepared using round punches and screened through sieve with sieve openings 1.0 mm on Erweka. After addition of 6 mg of magnesium stearate, the tabletting mixture was compressed on automatic rotary compressing machine Pressima into round-shaped cores with theoretical weight 150 mg at main pressure 10.8 kN. Hardness of the cores (performed on Erweka) was 38-58 N (average of N=20 was 48 N) and disintegration time (purified water, 37°C) 9 minutes.

### Example 22 (Reference)

20 g of solifenacin succinate, 259 g of lactose monohydrate and 18 g of povidone K25 were screened through the sieve with sieve openings 0.6 mm and homogeneously mixed.

Finally, 3 g of magnesium stearate was added to prepare tabletting mixture. Tabletting mixture was compressed on automatic rotary compressing machine Pressima into round-shaped cores with theoretical weight 150 mg at main pressure 9.5 kN. Hardness of the cores (performed on Erweka) was 49-64 N (average of N=20 was 56 N) and disintegration time (purified water, 37°C) 7-7.5 minutes.

Cores were coated in automatic coating machine Manesty XL with water-based film coating suspension, containing hydroxypropylcellulose, (62% by weight), titanium dioxide (13.5% by weight), talc (18.7% by weight), triacetin (5.8% by weight) and iron oxide (<0.05% by weight, not included in total sum of the dry constituents of the film coating suspension). The theoretical weight of the film coated tablets was 154 mg.

### Example 23 (Reference)

20 g of solifenacin succinate, 265 g of lactose monohydrate and 12 g of povidone K90F were screened through the sieve with sieve openings 0.6 mm and homogeneously mixed.

Finally, 3 g of magnesium stearate was added to prepare tabletting mixture. Tabletting mixture was compressed on automatic rotary compressing machine Pressima into round-shaped cores with theoretical weight 150 mg at main pressure 9.6 kN. Hardness of the cores (performed on Erweka) was 38-53 N (average of N=20 was 46 N) and disintegration time (purified water, 37°C) 2-2.5 minutes.

Cores were coated in automatic coating machine Manesty XL with water-based film coating suspension, containing hydroxypropylcellulose, (62% by weight), titanium dioxide (13.5% by weight), talc (18.7% by weight), triacetin (5.8% by weight) and iron oxide (<0.05% by weight, not included in total sum of the dry constituents of the film coating suspension). The theoretical weight of the film coated tablets was 154 mg.

### Example 24 (Reference)

20 g of solifenacin succinate, 235 g of lactose monohydrate, 30 g of pregelatinied starch and 12 g of povidone K25 were screened through the sieve with sieve openings 0.6 mm and homogeneously mixed.

Finally, 3 g of magnesium stearate was added to prepare tabletting mixture. Tabletting mixture was compressed on automatic rotary compressing machine Pressima into round-shaped cores with theoretical weight 150 mg at main pressure 10.6 kN. Hardness of the cores (performed on Erweka) was 40-48 N (average of N=20 was 43 N) and disintegration time (purified water, 37°C) 2 minutes.

Cores were coated in automatic coating machine Manesty XL with water-based film coating suspension, containing hydroxypropylcellulose, (62% by weight), titanium dioxide (13.5% by weight), talc (18.7% by weight), triacetin (5.8% by weight) and iron oxide (<0.05% by weight, not included in total sum of the dry constituents of the film coating suspension). The theoretical weight of the film coated tablets was 154 mg.

### Example 25 (Reference)

20 g of solifenacin succinate, 193 g of lactose monohydrate, 60 g of starch and 24 g of povidone K25 were screened through the sieve with sieve openings 0.6 mm and homogeneously mixed.

Finally, 3 g of magnesium stearate was added to prepare tabletting mixture. Tabletting mixture was compressed on automatic rotary compressing machine Pressima into round-shaped cores with theoretical weight 150 mg at main pressure 12.5 kN. Hardness of the cores (performed on Erweka) was 28-51 N (average of N=20 was 42 N) and disintegration time (purified water, 37°C) 4 minutes.

Cores were coated in automatic coating machine Manesty XL with water-based film coating suspension, containing hydroxypropylcellulose, (62% by weight), titanium dioxide (13.5% by weight), talc (18.7% by weight), triacetin (5.8% by weight) and iron oxide (<0.05% by weight, not included in total sum of the dry constituents of the film coating suspension). The theoretical weight of the film coated tablets was 154 mg.

### Example 26 (Reference)

20 g of solifenacin succinate, 245 g of lactose monohydrate, 20 g of starch and 12 g of povidone K25 were screened through the sieve with sieve openings 0.6 mm and homogeneously mixed.

Finally, 3 g of magnesium stearate was added to prepare tabletting mixture. Tabletting mixture was compressed on automatic rotary compressing machine Pressima into round-shaped cores with theoretical weight 150 mg at main pressure 10.5 kN. Hardness of the cores (performed on Erweka) was 52-60 N (average of N=20 was 57 N) and disintegration time (purified water, 37°C) 3.5 minutes.

Cores were coated in automatic coating machine Manesty XL with water-based film coating suspension, containing hydroxypropylcellulose, (62% by weight), titanium dioxide (13.5% by weight), talc (18.7% by weight), triacetin (5.8% by weight) and iron oxide (<0.05% by weight, not included in total sum of the dry constituents of the film coating suspension). The theoretical weight of the film coated tablets was 154 mg.

### Example 27 (Reference)

20 g of solifenacin succinate, 233 g of lactose monohydrate, 20 g of starch and 24 g of povidone K25 were screened through the sieve with sieve openings 0.6 mm and homogeneously mixed.

Finally, 3 g of magnesium stearate was added to prepare tabletting mixture. Tabletting mixture was compressed on automatic rotary compressing machine Pressima into round-shaped cores with theoretical weight 150 mg at main pressure 10.8 kN. Hardness of the cores (performed on Erweka) was 51-61 N (average of N=20 was 56 N) and disintegration time (purified water, 37°C) 4 minutes.

Cores were coated in automatic coating machine Manesty XL with water-based film coating suspension, containing hydroxypropylcellulose, (62% by weight), titanium dioxide (13.5% by weight), talc (18.7% by weight), triacetin (5.8% by weight) and iron oxide (<0.05% by weight, not included in total sum of the dry constituents of the film coating suspension). The theoretical weight of the film coated tablets was 154 mg.

### Example 28

The cores with the composition of the Example 1 were divided into four equal parts to be coated with four different water-based film coating suspensions with the following compositions:
- Composition of film coating suspension 1: hydroxypropylcellulose, (62% by weight), titanium dioxide (13.5% by weight), talc (18.7% by weight), triethyl citrate (5.8% by weight) and iron oxide (<0.05% by weight, not included in total sum of the dry constituents of the film coating suspension);
- Composition of film coating suspension 2: ready-to-use mixture Opadry 80W34100 AMB pink;
- Composition of film coating suspension 3: ready-to-use mixture Kollicoat IR white;
- Composition of film coating suspension 4: Eudragit E PO (62.5% by weight),, stearic acid (10% by weight), sodium laurylsulphate (Texapon K12) (15% by weight) and titanium dioxide (12.5% by weight).

The theoretical weight of the film coated tablets was 154 mg.

### Example 29 (Reference)

20 g of solifenacin succinate, 265 g of lactose monohydrate and 12 g of Kollicoat IR were screened through the sieve with sieve openings 0.6 mm and homogeneously mixed.

Finally, 3 g of magnesium stearate was added to prepare tabletting mixture. Tabletting mixture was compressed on automatic rotary compressing machine Pressima into round-shaped cores with theoretical weight 150 mg at main pressure 8.6 kN. Hardness of the cores (performed on Erweka) was 42-58 N (average of N=20 was 51 N) and disintegration time (purified water, 37°C) 1.5 minutes.

Cores were coated in automatic coating machine Manesty XL with water-based film coating suspension, containing hydroxypropylcellulose, (62% by weight), titanium dioxide (13.5% by weight), talc (18.7% by weight), triacetin (5.8% by weight) and iron oxide (<0.05% by weight, not included in total sum of the dry constituents of the film coating suspension). The theoretical weight of the film coated tablets was 154 mg.

### Example 30 (Reference)

20 g of solifenacin succinate, 265 g of lactose monohydrate and 12 g of polyvinyl alcohol were screened through the sieve with sieve openings 0.6 mm and homogeneously mixed.

Finally, 3 g of magnesium stearate was added to prepare tabletting mixture. Tabletting mixture was compressed on automatic rotary compressing machine Pressima into round-shaped cores with theoretical weight 150 mg at main pressure 10.2 kN. Hardness of the cores (performed on Erweka) was 25-44 N (average of N=20 was 34 N) and disintegration time (purified water, 37°C) 2.5 minutes.

Cores were coated in automatic coating machine Manesty XL with water-based film coating suspension, containing hydroxypropylcellulose, (62% by weight), titanium dioxide (13.5% by weight), talc (18.7% by weight), triacetin (5.8% by weight) and iron oxide (<0.05% by weight, not included in total sum of the dry constituents of the film coating suspension). The theoretical weight of the film coated tablets was 154 mg.

### Example 31 (Reference)

20 g of solifenacin succinate, 265 g of lactose monohydrate and 12 g of Opadry 80W34100 AMB pink were screened through the sieve with sieve openings 0.6 mm and homogeneously mixed.

Finally, 3 g of magnesium stearate was added to prepare tabletting mixture. Tabletting mixture was compressed on automatic rotary compressing machine Pressima into round-shaped cores with theoretical weight 150 mg at main pressure 8.5 kN. Hardness of the cores (performed on Erweka) was 45-57 N (average of N=20 was 51 N) and disintegration time (purified water, 37°C) 1.5-2 minutes.

Cores were coated in automatic coating machine Manesty XL with water-based film coating suspension, containing hydroxypropylcellulose, (62% by weight), titanium dioxide (13.5% by weight), talc (18.7% by weight), triacetin (5.8% by weight) and iron oxide (<0.05% by weight, not included in total sum of the dry constituents of the film coating suspension). The theoretical weight of the film coated tablets was 154 mg.

### Example 32 (Reference)

20 g of solifenacin succinate, 265 g of lactose monohydrate and 12 g of Eudragit E PO were screened through the sieve with sieve openings 0.6 mm and homogeneously mixed.

Finally, 3 g of magnesium stearate was added to prepare tabletting mixture. Tabletting mixture was compressed on automatic rotary compressing machine Pressima into round-shaped cores with theoretical weight 150 mg at main pressure 7.5 kN. Hardness of the cores (performed on Erweka) was 51-65 N (average of N=20 was 57 N) and disintegration time (purified water, 37°C) 4.5-5 minutes.

Cores were coated in automatic coating machine Manesty XL with water-based film coating suspension, containing hydroxypropylcellulose, (62% by weight), titanium dioxide (13.5% by weight), talc (18.7% by weight), triacetin (5.8% by weight) and iron oxide (<0.05% by weight, not included in total sum of the dry constituents of the film coating suspension). The theoretical weight of the film coated tablets was 154 mg.

### Example 33 (Reference)

175 g of solifenacin succinate, 4.81 kg of lactose monohydrate (commercially available as Tablettose 100) and povidone K25 were screened through a sieve with a sieve openings 0.6 mm and homogeneously mixed in a biconic mixer.

Finally, 53 g of magnesium stearate was added to prepare tabletting mixture. Tabletting mixture was compressed on automatic rotary compressing machine Kilian T100 into round-shaped cores with theoretical weight 150 mg at main pressure 12.0 kN. Hardness of the cores (performed on Erweka) was 72-84 N (average of N=20 was 78 N) and disintegration time (purified water, 37°C) 8-9 minutes.

Cores were coated in automatic coating machine Manesty XL with water-based film coating suspension, containing hydroxypropylcellulose, (62% by weight), titanium dioxide (13.5% by weight), talc (18.7% by weight) and triacetin (5.8% by weight). The theoretical weight of the film coated tablets was 154 mg.

### Example 34 (Reference)

175 g of solifenacin succinate, 4.81 kg of lactose monohydrate (commercially available as FlowLac 100) and povidone K25 were screened through a sieve with a sieve openings 0.6 mm and homogeneously mixed in a biconic mixer.

Finally, 53 g of magnesium stearate was added to prepare tabletting mixture. Tabletting mixture was compressed on automatic rotary compressing machine Kilian T100 into round-shaped cores with theoretical weight 150 mg at main pressure 8.7 kN. Hardness of the cores (performed on Erweka) was 91-108 N (average of N=20 was 100 N) and disintegration time (purified water, 37°C) 8 minutes.

Cores were coated in automatic coating machine Manesty XL with water-based film coating suspension, containing hydroxypropylcellulose, (62% by weight), titanium dioxide (13.5% by weight), talc (18.7% by weight) and triacetin (5.8% by weight). The theoretical weight of the film coated tablets was 154 mg.

### Example 35 (Reference)

175 g of solifenacin succinate, 4.81 kg of lactose monohydrate (commercially available as SuperTab 14SD) and povidone K25 were screened through a sieve with a sieve openings 0.6 mm and homogeneously mixed in a biconic mixer.

Finally, 53 g of magnesium stearate was added to prepare tabletting mixture. Tabletting mixture was compressed on automatic rotary compressing machine Kilian T100 into round-shaped cores with theoretical weight 150 mg at main pressure 8.7 kN. Hardness of the cores (performed on Erweka) was 89-106 N (average of N=20 was 98 N) and disintegration time (purified water, 37°C) 8 minutes.

Cores were coated in automatic coating machine Manesty XL with water-based film coating suspension, containing hydroxypropylcellulose, (62% by weight), titanium dioxide (13.5% by weight), talc (18.7% by weight) and triacetin (5.8% by weight). The theoretical weight of the film coated tablets was 154 mg.

### Comparative Example 1

20 g of solifenacin succinate, 205 g of lactose monohydrate (particles B) (table 2) and 60 g of maize starch were screened through the sieve with sieve openings 0.6 mm and homogeneously mixed in a chamber of a fluid-bed granulator at inlet air temperature 55°C up to the temperature of a homogenized mixture 35.6°C. The homogenized mixture was granulated in a fluid-bed granulator with 10% w/w water dispersion of hydroxypropylmethylcellulose (the granulating liquid was comprised of 12 g of hydroxypropylmethylcellulose and 108 g of purified water) at inlet air temperature 55-60°C and pump rate 7-10 rpm. The temperature of the wet granulate at the end of spraying was 26.1°C. The drying was performed at inlet air temperature 55-60°C for 7 minutes up to the temperature of the granulate 36.7°C. The granulate was sieved through the sieve with sieve openings 1.0 mm. Loss on drying of the granulate (measured using a Mettler Toledo HR73 halogen moisture analyzer at 85°C for 20 minutes) was 2.00%.

Finally, 3 g of magnesium stearate was added to prepare tabletting mixture. Loss on drying of the tabletting mixture (measured using a Mettler Toledo HR73 halogen moisture analyzer at 85°C for 20 minutes) was 2.10%.

The tabletting mixture was compressed on automatic rotary compressing machine Pressima into round-shaped cores with theoretical weight of 150 mg. Hardness of the cores (performed on Erweka) was 30-69 N (average of n = 20 cores was 56 N) and disintegration time (purified water, 37°C) 7 minutes.

Cores were coated according to a process as described in Example 1. Hardness of the film coated tablets (performed on Erweka) was 59-91 N (average of n = 10 was 76 N).

### Comparative Example 2

20 g of solifenacin succinate, 205 g of lactose monohydrate (particles B) (table 2A) and 60 g of maize starch were screened through the sieve with sieve openings 0.6 mm and homogeneously mixed in a chamber of a high-shear mixer for 1 minute at speed of impeller 1000 rpm. The homogenized mixture was granulated in a chamber of high-shear mixer with water dispersion of hydroxypropylmethylcellulose (the granulating dispersion was comprised of 12 g of hydroxypropylmethylcellulose and 54 g of purified water) for 1 minute at speed of impeller 500 rpm and aped of chopper 100 rpm. The wet granulate was transfered into a chamber of a fluid-bed dryer wherein the drying process was performed at inlet air temperature 50°C for 8 minutes up to the temperature of the granulate 36.1°C. The granulate was sieved through the sieve with sieve openings 1.0 mm. Loss on drying of the granulate (measured using a Mettler Toledo HR73 halogen moisture analyzer at 85°C for 20 minutes) was 1.60%.

Finally, 3 g of magnesium stearate was added to prepare tabletting mixture. Loss on drying of the tabletting mixture (measured using a Mettler Toledo HR73 halogen moisture analyzer at 85°C for 20 minutes) was 1.58%.

The tabletting mixture was compressed on automatic rotary compressing machine Pressima into round-shaped cores with theoretical weight of 150 mg. Hardness of the cores (performed on Erweka) was 43-65 N (average of n = 20 cores was 56 N) and disintegration time (purified water, 37°C) 6 minutes.

Cores were coated according to a process as described in Example 1. Hardness of the film coated tablets (performed on Erweka) was 58-84 N (average of n=10 was 72 N).

### Comparative Example 3

33.53 g of solifenacin hydrogen sulphate, 360.22 g of lactose monohydrate (particles B) (table 2A) and 105 g of maize starch were screened through the sieve with sieve openings 0.6 mm and homogeneously mixed in a chamber of a fluid-bed granulator at inlet air temperature 55°C up to the temperature of a homogenized mixture 35.1°C for 4 minutes. The homogenized mixture was granulated in a fluid-bed granulator with water dispersion of hydroxypropylmethylcellulose (the granulating liquid was comprised of 21 g of hydroxypropylmethylcellulose and 219 g of purified water) at inlet air temperature 55°C and pump rate 5-8 rpm for 10 minutes. The temperature of the wet granulate at the end of spraying was 27.1°C. The drying was performed at inlet air temperature 55°C for 4 minutes up to the temperature of the granulate 36.7°C. The granulate was sieved through the sieve with sieve openings 1.0 mm. Loss on drying of the granulate (measured using a Mettler Toledo HR73 halogen moisture analyzer at 105°C for 5 minutes) was 1.28%.

Finally, 5.25 g of magnesium stearate was added to prepare tabletting mixture.

The tabletting mixture was compressed on automatic rotary compressing machine Pressima at the main pressure 6.2 kN into round-shaped cores with theoretical weight of 150 mg. Hardness of the cores (performed on Erweka) was 48-62 N and disintegration time (purified water, 37°C) 9 minutes.

Cores were coated according to a process as described in Example 1.

### Results of stress stability testing

The stress stability testing of the formulations containing solifenacin succinate was perfomed by HPLC after storage at 40°C/75% relative humidity (RH) (closed) for 5 weeks. We compared the formulation, prepared by direct compression (Example 1) with the formulation prepared by wet granulation process. Furthermore, the wet granulation process was performed in two ways, i.e. by fluid-bed granulation (Comparative Example 1) and by high-shear-granulation (Comparative Example 2) at initial time (t=0) and after exposure to 40°C/75% RH (closed) for 5 weeks. Results are presented as amount of impurity N-oxide of solifenacin succinate at Rt=10.6 min (table 15).

**Table 15. Results of stress stability study - amount of impurity N-oxide of solifenacin succinate.**

| Time of testing | Example 1 | Comparative Example 1 | Comparative Example 2 |
|---|---|---|---|
| Initial time (% area) | 0.03 | 0.07 | 0.06 |
| After storing at 40°C/75% RH (closed) for 5 weeks (% area) | 0.09 | 0.23 | 0.22 |

As can be seen, both absolute amount of the impurity N-oxide of solifenacin succinate at initial time and after exposure to 40°C/75% RH (closed) for 5 weeks of the Example 1 is significantly smaller if compared to Comparative Example 1 and Comparative Example 2 at both initial times (0.03 area %, 0.07 area % and 0.06 area % for Example 1, Comparative Example 1 and Comparative Example 2, subsequently) and after 5 weeks (0.09 area %, 0.23 area % and 0.22 area % for Example 1, Comparative Example 1 and Comparative Example 2, subsequently). Furthermore, also relative increase of the amount of the impurity N-oxide of solifenacin succinate from initial time to 5 weeks after exposure to 40°C/75% RH (closed) is significantly smaller in Example 1 when comparing Comparative Example 1 and Comparative Example 2 (0.06 area %, 0.16 area % and 0.16 area % for Example 1, Comparative Example 1 and Comparative Example 2, subsequently).

The results of stress stability study unambiguously confirms the advantage of the dry process i.e. in the absence of a granulating liquid as presented in Example 1 in comparison to the wet granulation process as presented in Comparative Example 1 and Comparative Example 2.

The stress stability testing of the formulations containing solifenacin hydrogensulphate was perfomed by HPLC after storage at 40°C/75% relative humidity (RH) (closed) for 2 weeks. We compared different formulations, prepared by direct compression (Examples 16, 17 and 19) with the formulation prepared by wet granulation process (Comparative Example 3). Results are presented as amount of oxidation product of solifenacin hydrogensulphate at Rt=12.3 min (table 16).

**Table 16. Results of stress stability study - amount of oxidation product of solifenacin hydrogen sulphate.**

| Time of testing | Example 16 | Example 17 | Example 19 | Comparative Example 3 |
|---|---|---|---|---|
| After storing at 40°C/75% RH (closed) for 2 weeks (% area) | 0.06 | 0.03 | 0.03 | 0.12 |

As can be seen, both absolute amount of the oxidation product of solifenacin hydrogensulphate after exposure to 40°C/75% RH (closed) for 2 weeks of the Examples 16, 17 and 19 are significantly smaller if comparing to Comparative Example 3_(0.06 area %, 0.03 area %, 0.03 are % and 0.06 area % for Examples 16, 17 and 19 and Comparative Example 3, subsequently).

The results of stress stability study unambiguously confirm the advantage of the dry process i.e. in the absence of a granulating liquid as presented in Examples 16, 17 and 19 in comparison to the wet granulation process as presented in Comparative Example 3.

### Results of dissolution studies

The disitegration time of film coated tablets prepared according to Example 1, Comparative Example 1 and Comparative Example 2 in artificial gastric juice pH 2.0 and phosphate buffer pH 6.8 was compared (table 17).

**Table 17. Disintegration time of film coated tablets in artificial gastric juice pH 2.0 and phosphate buffer pH 6.8.**

| Test | Example 1 | Comparative Example 1 | Comparative Example 2 |
|---|---|---|---|
| Disintegration time in artificial gastric juice pH 2.0 (minutesaeconds) | 3:28 | 7:56 | 5:36 |
| Disintegration time in phosphate buffer pH 6.8 (minutes:seconds) | 4:07 | 6:54 | 5:21 |

As can be seen, disintegration time of both Comparative Example 1 and Comparative Example 2 is significantly longer than in Example 1 in both media, i.e. in artificial gastric juice pH 2.0 and phosphate buffer pH 6.8, which was further confirmed in dissolution profile in artificial gastric juice pH 2.0 of the two examples, i.e. Example 1 and Comparative Example 1.

The percentages of dissolved solifenacin in film coated tablets of Example 1 and Comparative Example 1 in artificial gastric juice pH 2.0 are collected in table 18.

**Table 18. Percentage of dissolved solifenain from film coated tablets of Example 1, 3, 24 and Comparative Example 1 in artificial gastric juice pH 2.0.**

| Time (min) | Example 1 % dissolved solifenacin | Example 3 % dissolved solifenacin | Example 24 % dissolved solifenacin | Comparative Example 1 % dissolved solifenacin |
|---|---|---|---|---|
| 0 | 0 | 0 | 0 | 0 |
| 10 | 31 | 92 | 94 | 19 |
| 15 | 50 | 95 | 100 | 28 |
| 30 | 66 | 97 | 101 | 56 |
| 45 | 87 | 98 | 101 | 84 |
| 60 | 99 | 98 | 100 | 98 |

As can be seen, faster disintegration of film coated tablets of Example 1 (table 17) was confirmed by dissolution profile up to 45 minutes, wherein dissolution rate of Examples 1,3 and 24 is higher than of Comparative Example 1.

## Claims

1. A process for the preparation of a solid oral dosage form comprising:
a.) an effective amount of crystalline solifenacin succinate,
b.) pharmaceutically acceptable additives suitable for the preparation of solid oral dosage forms,
in the absence of a solvent,
said solid oral dosage form prepared by a process in the absence of a solvent, comprising one or more disintegrants and/or superdisintegrants in the range 1 to 90% by weight, preferably 1-40% by weight, more preferably 1-25% by weight, a binder in the amount 1 to 90% by weight, preferably 1-50% by weight, a lubricant in the amount 0.1 to 10% by weight, and a filler or diluent within a range 20-99% by weight, preferably 50-99% by weight, so that the total sum of the combination of components of the formulation is 100%,
the main excipient being lactose monohydrate, microcrystalline cellulose or mannitol, and
pharmaceutical excipients being used in which, cumulatively, the average particle size of the main excipient is larger than 60 µm.

2. A process for the preparation of solid dosage forms according to claim 1, comprising the steps of:
i) optionally grinding crystalline solifenacin succinate and pharmaceutically acceptable excipients,
ii) subjecting a homogenous mixture of the optionally ground active ingredient and excipients to compression to form a comprimate,
iii) converting the comprimate to form a granulate and
iv) compressing/ tabletting the granulate to form the solid oral dosage form.

3. A process according to claims 1 or 2, **characterized in that** it is a roller compaction method.

4. A process for the preparation of solid dosage forms according to claim 1, comprising the steps of
a.) homogenously mixing crystalline solifenacin succinate with pharmaceutically acceptable excipients,
b.) compressing! tabletting of the mixed active ingredient and pharmaceutically acceptable excipients to form a solid dosage form.

5. A process for the preparation of solid dosage forms according to any of claims 1 to 4, **characterized in that** pharmaceutical excipients are used in which, cumulatively, the average particle size of the main excipient is larger than 70 µm, more preferably larger than 80 µm, most preferably larger than 90 µm.

6. A process for the preparation of solid dosage forms according to any of claims 1 to 5, **characterized in that** pharmaceutical excipients are used in which the angle of repose of the main excipient is below 36°, preferably below 34°, most preferably below 32°.

7. A process for the preparation of solid dosage forms according to any of claims 1 to 6, **characterized in that** pharmaceutical excipients are used in which the Hausner ratio of the main excipient is below 1.40, preferably below 1.30, more preferably below 1.28, most preferably below 1.26.

8. A solid oral dosage form prepared by a process in the absence of a solvent, comprising
a.) an effective amount of crystalline solifenacin succinate,
b.) pharmaceutically acceptable additives suitable for the preparation of solid oral dosage forms,
said solid oral dosage form prepared by a process in the absence of a solvent, comprising one or more disintegrants and/or superdisintegrants in the range 1 to 90% by weight, preferably 1-40% by weight, more preferably 1-25% by weight, a binder in the amount 1 to 90% by weight, preferably 1-50% by weight, a lubricant in the amount 0.1 to 10% by weight, and a filler or diluent within a range 20-99% by weight, preferably 50-99% by weight, so that the total sum of the combination of components of the formulation is 100%,
the main excipient being lactose monohydrate, microcrystalline cellulose or mannitol, and average particle size of the main excipient being larger than 60 µm,
said process being a process for the preparation of a solid oral dosage form according to claim 1 and said process being a process selected from the group consisting of a process for the preparation of solid dosage forms comprising the steps of:
i) optionally grinding crystalline solifenacin succinate and pharmaceutically acceptable excipients,
ii) subjecting a homogenous mixture of the optionally ground active ingredient and excipients to compression to form a comprimate,
iii) converting the comprimate to form a granulate and
iv) compressing! tabletting the granulate to form the solid oral dosage form,
a process for the preparation of a solid oral dosage form, which is a roller compaction method, and
a process for the preparation of solid dosage forms comprising the steps of:
a.) homogenously mixing crystalline solifenacin succinate with pharmaceutically acceptable excipients,
b.) compressing/ tabletting of the mixed active ingredient and pharmaceutically acceptable excipients to form a solid dosage form.

9. A solid oral dosage form according to claim 8, **characterized in that** it is prepared by direct compression or roller compaction.

10. A solid oral dosage form according to any of claims 8 to 9, **characterized in that** average particle size of the main excipient is larger than 70 µm, more preferably larger than 80 µm, most preferably larger than 90 µm.

11. Solid oral dosage form according to any of claims 8 to 10, for use in the treatment of, alleviation and/or prevention of overactive bladder syndrome, urinary urgency and increased urinary frequency.

## Patentansprüche

1. Verfahren zur Herstellung einer festen oralen Dosierungsform, umfassend:
a.) eine wirksame Menge von kristallinem Solifenacinsuccinat,
b.) pharmazeutisch unbedenkliche Zusatzstoffe, die sich für die Herstellung von festen oralen Dosierungsformen eignen,
in Abwesenheit eines Lösungsmittels,
wobei die feste orale Dosierungsform durch ein Verfahren in Abwesenheit eines Lösungsmittels hergestellt wird, umfassend ein oder mehrere Sprengmittel und/oder Supersprengmittel im Bereich von 1 bis 90 Gew.-%, vorzugsweise 1-40 Gew.-%, besonders bevorzugt 1-25 Gew.-%, ein Bindemittel in einer Menge von 1 bis 90 Gew.-%, vorzugsweise 1-50 Gew.-%, ein Schmiermittel in einer Menge von 0,1 bis 10 Gew.-% und einen Füllstoff oder ein Verdünnungsmittel im Bereich von 20-99 Gew.-%, vorzugsweise 50-99 Gew.-%, so dass die Gesamtsumme der Kombination an Komponenten der Formulierung 100% beträgt, wobei es sich bei dem Hauptexzipienten um Lactosemonohydrat, mikrokristalline Cellulose oder Mannitol handelt, und
wobei pharmazeutische Exzipienten verwendet werden, bei denen kumulativ die durchschnittliche Teilchengröße des Hauptexzipienten größer als 60 µm ist.

2. Verfahren zur Herstellung der festen Dosierungsformen nach Anspruch 1, welches die folgenden Schritte umfasst:
i) gegebenenfalls Mahlen von kristallinem Solifenacinsuccinat und pharmazeutisch unbedenklichen Exzipienten,
ii) Verpressen einer homogenen Mischung des gegebenenfalls gemahlenen Wirkstoffs und der Exzipienten unter Bildung eines Komprimats,
iii) Konvertieren des Komprimats unter Bildung eines Granulats und
iv) Komprimieren/Tablettieren des Granulats unter Bildung der festen oralen Dosierungsform.

3. Verfahren nach Anspruch 1 oder 2, **dadurch gekennzeichnet, dass** es sich um ein Walzenverdichtungsverfahren handelt.

4. Verfahren zur Herstellung der festen Dosierungsformen nach Anspruch 1, welches die folgenden Schritte umfasst:
a.) das homogene Mischen von kristallinem Solifenacinsuccinat mit pharmazeutisch unbedenklichen Exzipienten,
b.) das Komprimieren/Tablettieren des gemischten Wirkstoffs und der pharmazeutisch unbedenklichen Exzipienten unter Bildung einer festen Dosierungsform.

5. Verfahren zur Herstellung der festen Dosierungsformen nach einem der Ansprüche 1 bis 4, **dadurch gekennzeichnet, dass** man pharmazeutische Exzipienten verwendet, bei denen kumulativ die durchschnittliche Teilchengröße des Hauptexzipienten größer als 70 µm, besonders bevorzugt größer als 80 µm, am meisten bevorzugt größer als 90 µm ist.

6. Verfahren zur Herstellung der festen Dosierungsformen nach einem der Ansprüche 1 bis 5, **dadurch gekennzeichnet, dass** man pharmazeutische Exzipienten verwendet, bei denen der Böschungswinkel des Hauptexzipienten unter 36°, vorzugsweise unter 34°, besonders bevorzugt unter 32° liegt.

7. Verfahren zur Herstellung der festen Dosierungsformen nach einem der Ansprüche 1 bis 6, **dadurch gekennzeichnet, dass** man pharmazeutische Exzipienten verwendet, bei denen das Hausner-Verhältnis des Hauptexzipienten unter 1,40, vorzugsweise unter 1,30, besonders bevorzugt unter 1,28, am meisten bevorzugt unter 1,26 liegt.

8. Feste orale Dosierungsform, hergestellt durch ein Verfahren in Abwesenheit eines Lösungsmittels, umfassend
a.) eine wirksame Menge von kristallinem Solifenacinsuccinat,
b.) pharmazeutisch unbedenkliche Zusatzstoffe, die sich für die Herstellung von festen oralen Dosierungsformen eignen,
wobei die feste orale Dosierungsform durch ein Verfahren in Abwesenheit eines Lösungsmittels hergestellt wird, umfassend ein oder mehrere Sprengmittel und/oder Supersprengmittel im Bereich von 1 bis 90 Gew.-%, vorzugsweise 1-40 Gew.-%, besonders bevorzugt 1-25 Gew.-%, ein Bindemittel in einer Menge von 1 bis 90 Gew.-%, vorzugsweise 1-50 Gew.-%, ein Schmiermittel in einer Menge von 0,1 bis 10 Gew.-% und einen Füllstoff oder ein Verdünnungsmittel im Bereich von 20-99 Gew.-%, vorzugsweise 50-99 Gew.-%, so dass die Gesamtsumme der Kombination an Komponenten der Formulierung 100% beträgt, wobei es sich bei dem Hauptexzipienten um Lactosemonohydrat, mikrokristalline Cellulose oder Mannitol handelt, und
wobei die durchschnittliche Teilchengröße des Hauptexzipienten größer als 60 µm ist,
wobei es sich bei dem Verfahren um ein Verfahren zur Herstellung einer festen oralen Dosierungsform nach Anspruch 1 handelt, und es sich bei dem Verfahren um ein Verfahren ausgewählt aus der Gruppe bestehend aus einem Verfahren zur Herstellung von festen Dosierungsformen, welches die folgenden Schritte umfasst:
i) gegebenenfalls Mahlen von kristallinem Solifenacinsuccinat und pharmazeutisch unbedenklichen Exzipienten,
ii) Verpressen einer homogenen Mischung des gegebenenfalls gemahlenen Wirkstoffs und der Exzipienten unter Bildung eines Komprimats,
iii) Konvertieren des Komprimats unter Bildung eines Granulats und
iv) Komprimieren/Tablettieren des Granulats unter Bildung der festen oralen Dosierungsform,
einem Verfahren zur Herstellung einer festen oralen Dosierungsform, bei dem es sich um ein Walzenverdichtungsverfahren handelt, und
einem Verfahren zur Herstellung von festen Dosierungsformen, welches die folgenden Schritte umfasst:
a.) das homogene Mischen von kristallinem Solifenacinsuccinat mit pharmazeutisch unbedenklichen Exzipienten,
b.) das Komprimieren/Tablettieren des gemischten Wirkstoffs und der pharmazeutisch unbedenklichen Exzipienten unter Bildung einer festen Dosierungsform
handelt.

9. Feste orale Dosierungsform nach Anspruch 8, **dadurch gekennzeichnet, dass** sie durch direktes Verpressen oder Walzenverdichtung hergestellt wird.

10. Feste orale Dosierungsform nach einem der Ansprüche 8 bis 9, **dadurch gekennzeichnet, dass** die durchschnittliche Teilchengröße des Hauptexzipienten größer als 70 µm, besonders bevorzugt größer als 80 µm, am meisten bevorzugt größer als 90 µm ist.

11. Feste orale Dosierungsform nach einem der Ansprüche 8 bis 10 zur Verwendung bei der Behandlung von, Linderung und/oder Prävention von einer überaktiven Blase, Harndrang und Pollakisurie.

## Revendications

1. Procédé de préparation d'une forme posologique orale solide comprenant :
a.) une quantité efficace de succinate de solifénacine cristallin,
b.) des additifs pharmaceutiquement acceptables adaptés à la préparation de formes posologiques solides orales,
en l'absence d'un solvant,
ladite forme posologique orale solide préparée par un procédé en l'absence d'un solvant, comprenant un ou plusieurs délitants et/ou superdélitants dans la plage de 1 à 90 % en poids, de préférence de 1 à 40 % en poids, plus préférablement de 1 à 25 % en poids, un liant en la quantité de 1 à 90 % en poids, de préférence de 1 à 50 % en poids, un lubrifiant en la quantité de 0,1 à 10 % en poids, et une charge ou un diluant dans une plage de 20 à 99 % en poids, de préférence de 50 à 99 % en poids, de sorte que la somme totale de la combinaison de composants de la formulation soit de 100 %,
le principal excipient étant le lactose monohydraté, la cellulose microcristalline ou le mannitol, et des excipients pharmaceutiques étant utilisés, dans lesquels, cumulativement, la taille moyenne de particule du principal excipient est supérieure à 60 µm.

2. Procédé de préparation de formes posologiques solides selon la revendication 1, comprenant les étapes suivantes :
i) éventuellement le broyage de succinate de solifénacine cristallin et d'excipients pharmaceutiquement acceptables,
ii) l'exposition d'un mélange homogène de la substance active et des excipients éventuellement broyés à une compression pour former un comprimé,
iii) la conversion du comprimé pour former un granulé et
iv) la compression/mise en forme du granulé pour former la forme posologique orale solide.

3. Procédé selon les revendications 1 ou 2, **caractérisé en ce qu'**il s'agit d'un procédé de compactage au rouleau.

4. Procédé de préparation de formes posologiques solides selon la revendication 1, comprenant les étapes suivantes :
a.) le mélange homogène de succinate de solifénacine cristallin avec des excipients pharmaceutiquement acceptables,
b.) la compression/mise en forme de la substance active et des excipients pharmaceutiquement acceptables mélangés pour former une forme posologique solide.

5. Procédé de préparation de formes posologiques solides selon l'une quelconque des revendications 1 à 4, **caractérisé en ce que** des excipients pharmaceutiques sont utilisés, dans lesquels, cumulativement, la taille moyenne de particule du principal excipient est supérieure à 70 µm, plus préférablement supérieure à 80 µm, de manière préférée entre toutes supérieure à 90 µm.

6. Procédé de préparation de formes posologiques solides selon l'une quelconque des revendications 1 à 5, **caractérisé en ce que** des excipients pharmaceutiques sont utilisés, dans lesquels l'angle de repos du principal excipient est inférieur à 36°, de préférence inférieur à 34°, de manière préférée entre toutes inférieur à 32°.

7. Procédé de préparation de formes posologiques solides selon l'une quelconque des revendications 1 à 6, **caractérisé en ce que** des excipients pharmaceutiques sont utilisés, dans lesquels le rapport Hausner du principal excipient est inférieur à 1,40, de préférence inférieur à 1,30, plus préférablement inférieur à 1,28, de manière préférée entre toutes inférieur à 1,26.

8. Forme posologique orale solide préparée par un procédé en l'absence d'un solvant, comprenant
a.) une quantité efficace de succinate de solifénacine cristallin,
b.) des additifs pharmaceutiquement acceptables adaptés à la préparation de formes posologiques solides orales,
ladite forme posologique orale solide préparée par un procédé en l'absence d'un solvant, comprenant un ou plusieurs délitants et/ou superdélitants dans la plage de 1 à 90 % en poids, de préférence de 1 à 40 % en poids, plus préférablement de 1 à 25 % en poids, un liant en la quantité de 1 à 90 % en poids, de préférence de 1 à 50 % en poids, un lubrifiant en la quantité de 0,1 à 10 % en poids, et une charge ou un diluant dans une plage de 20 à 99 % en poids, de préférence de 50 à 99 % en poids, de sorte que la somme totale de la combinaison de composants de la formulation soit de 100 %,
le principal excipient étant le lactose monohydraté, la cellulose microcristalline ou le mannitol, et la taille moyenne de particule du principal excipient étant supérieure à 60 µm,
ledit procédé étant un procédé de préparation d'une forme posologique orale solide selon la revendication 1 et ledit procédé étant un procédé choisi dans le groupe constitué de un procédé de préparation de formes posologiques solides comprenant les étapes suivantes :
i) éventuellement le broyage de succinate de solifénacine cristallin et d'excipients pharmaceutiquement acceptables,
ii) l'exposition d'un mélange homogène de la substance active et des excipients éventuellement broyés à une compression pour former un comprimé,
iii) la conversion du comprimé pour former un granulé et
iv) la compression/mise en forme du granulé pour former la forme posologique orale solide,
un procédé de préparation d'une forme posologique orale solide, qui est un procédé de compactage au rouleau, et
un procédé de préparation de formes posologiques solides comprenant les étapes suivantes :
a.) le mélange homogène de succinate de solifénacine cristallin avec des excipients pharmaceutiquement acceptables,
b.) la compression/mise en forme de la substance active et des excipients pharmaceutiquement acceptables mélangés pour former une forme posologique solide.

9. Forme posologique orale solide selon la revendication 8, **caractérisée en ce qu'**elle est préparée par compression directe ou compactage au rouleau.

10. Forme posologique orale solide selon l'une quelconque des revendications 8 à 9, **caractérisée en ce que** la taille moyenne de particule du principal excipient est supérieure à 70 µm, plus préférablement supérieure à 80 µm, de manière préférée entre toutes supérieure à 90 µm.

11. Forme posologique orale solide selon l'une quelconque des revendications 8 à 10, pour utilisation dans le traitement, l'atténuation et/ou la prévention du syndrome de vessie hyperactive, de l'urgence urinaire et de la fréquence urinaire accrue.
